(19) Europäisches Patentamt
European Patent Office
Office européen des brevets

(11) Numéro de publication: 0 133 098
B1

(12) # FASCICULE DE BREVET EUROPÉEN

(45) Date de publication du fascicule du brevet: 14.01.87

(51) Int. Cl.⁴: C 07 K 7/00

(21) Numéro de dépôt: 84401479.5

(22) Date de dépôt: 12.07.84

(54) **Nouveaux dérivés de synergistines et leur préparation.**

(30) Priorité: 13.07.83 FR 8311706

(43) Date de publication de la demande: 13.02.85 Bulletin 85/7

(45) Mention de la délivrance du brevet: 14.01.87 Bulletin 87/3

(84) Etats contractants désignés: AT BE CH DE FR GB IT LI LU NL SE

(56) Documents cité:
US-A-4 355 112

PROCEEDINGS - AN INTERNATIONAL CONFERENCE ON TRENDS IN ANTIBIOTIC RESEARCH - GENETICS, BIOSYNTHESES, ACTIONS & NEW SUBSTANCES, 14-15 juin 1982, Tokyo, pages 89-98, Japan Antibiotics Research Association, Tokyo, JP; M.-L. CAPMAU et al.: "Mechanism of action of the pristinamycins"

CHEMICAL ABSTRACTS, vol. 71, no. 11, 1969, page 201, no. 47912e, Columbus, Ohio, US; M. DELEPINE: "Pristinamycin; an antibiotic with two synergystic components"

CHEMICAL ABSTRACTS, vol. 69, no. 3, 1968, page 1031, no. 10707z, Columbus, Ohio, US; J. PREUD'HOMME et al.: "Isolation, characterization, and identification of the components of pristinamycin"

(73) Titulaire: RHONE- POULENC SANTE, Les Miroirs 18 Avenue d'Alsace, F-92400 Courbevoie Cedex (FR)

(72) Inventeur: Corbet, Jean- Pierre, "Les Marronniers" Résidence "Charrière Blanche", F-69140 Ecully (FR)
Inventeur: Cotrel, Claude, 17A avenue du Docteur Arnold Netter, F-75012 Paris (FR)
Inventeur: Farge, Daniel, 30 rue des Pins Sylvestres, F-94320 Thiais (FR)
Inventeur: Paris, Jean- Marc, 8 rue des Acacias, F-77360 Vaires- sur- Marne (FR)

(74) Mandataire: Gaumont, Robert, RHONE- POULENC RECHERCHES Service Brevets Pharma 25, Quai Paul Doumer, F-92408 Courbevoie Cedex (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 133 098 B1

LIBER, STOCKHOLM 1987

**Description**

La pristinamycine et la virginismycine sont des produits connus: J. Preud'homme et coll., Bull. Soc. Chim. Fr, 2 585-91, (1968).

La présente invention concerne de nouveaux dérivés de synergistines de formule générale:

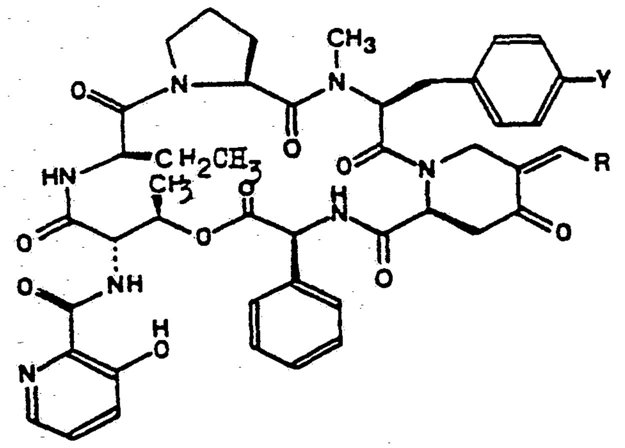

(I)

utiles comme intermédiaires pour la préparation d'autres dérivés de synergistines thérapeutiquement actifs. ainsi que leurs sels lorsqu'ils existent.

Dans la formule générale (I), Y représente un atome d'hydrogène ou un radical diméthylamino et R représente:

a) soit un atome d'hydrogène ou un radical hydroxy

b) soit un radical de formle générale:

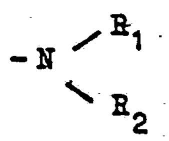

(II)

dans laquelle $R_1$ et $R_2$, identiques ou différents, représentent un atome d'hydrogène ou un radical phényle ou pyridyle éventuellement substitués (par un radical dialcoylamino dont la partie alcoyle contient 1 à 4 atomes de carbone en chaîne droite ou ramifiée) ou un radical alcoyle contenant 1 à 10 atomes de carbone en chaîne droite ou ramifiée éventuellement substitué (par un radical hydroxy, mercapto, carboxy, pyridyle, anilino, alcoylamino ou dialcoylamino dont au moins l'une des parties alcoyle est elle-même substituée par un radical hydroxy, mercapto, carboxy ou anilino), ou un radical alcényle contenant 3 ou 4 atomes de carbone, ou un radical alcynyle contenant 3 ou 4 atomes de carbone, ou bien $R_1$ et $R_2$ forment ensemble avec l'atome d'azote auquel ils sont liés un hétérocycle à 5 ou 6 chaînons contenant éventuellement un autre hétéroatome choisi parmi l'oxygène, le soufre ou l'azote (éventuellement substitué par un radical alcoyle),

c) soit un atome d'halogène ou un radical triméthylsilyloxy, dialcoylphosphoryloxy ou un radical de formule générale:

$- O\ SO_2\ R_3$ (IIa)

ou $- O\ CO\ R_4$ (IIb)

dans laquelle $R_3$ est un radical alcoyle, trifluorométhyle, trichlorométhyle, ou un radical phényle éventuellement substitué par un atome d'halogène ou par un radical alcoyle ou nitro et $R_4$ est défini comme $R_3$ ou représente un radical alcoylcarbonylméthyle, alcoylcarbonyl-2 éthyle, alcoyloxycarbonylméthyle, alcoyloxycarbonyl-2 éthyle ou alcoyloxy.

Il est entendu que les radicaux et portions alcoyle cités ci-dessus ou qui seront cités ci-après sont (sauf mention spéciale) droits ou ramifiés et contiennent 1 à 4 atomes de carbone.

Lorsque le radical R représente un atome d'halogène, il peut être choisi parmi les atomes de chlore et de brome.

Par ailleurs, il est également entendu que les produits de formule générale (I) dans laquelle R est autre qu'un radical de formule générale (II) peuvent se présenter sous 2 formes isomères et que ces isomères et leurs mélanges entrent dans le cadre de la présente invention.

Selon l'invention, les produits de formule générale (I) peuvent être préparés par action d'un produit de formule générale:

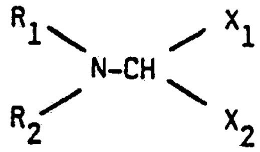

(III)

[[dans laquelle $R_1$ at $R_2$ sont des radicaux alcoyle contenant 1 à 4 atomes de carbone ou forment ensemble avec l'atome d'azote auquel ils sont liés un hétérocycle à 5 ou 6 chaînons tel que défini précédemment en b) et, $X_1$ et $X_2$, identiques ou différents, représentent un radical alcoyloxy ou un radical amino substitué défini comme $-NR_1R_2$]], sur un produit de formule générale:

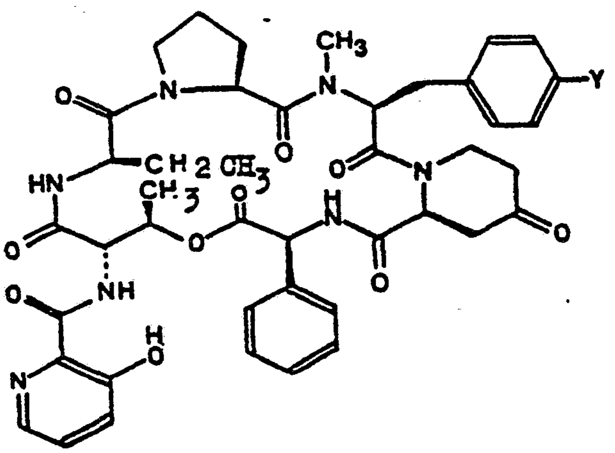

(IV)

dans laquelle Y est défini comme précédemment, pour obtenir un produit de formule générale (I) dans laquelle Y étant défini comme précédemment, R représente un radical de formule générale (II) dans lequel $R_1$ et $R_2$ ont la signification donnée ci-dessus pour la formule générale (III), c'est-à-dire, un produit de formule générale

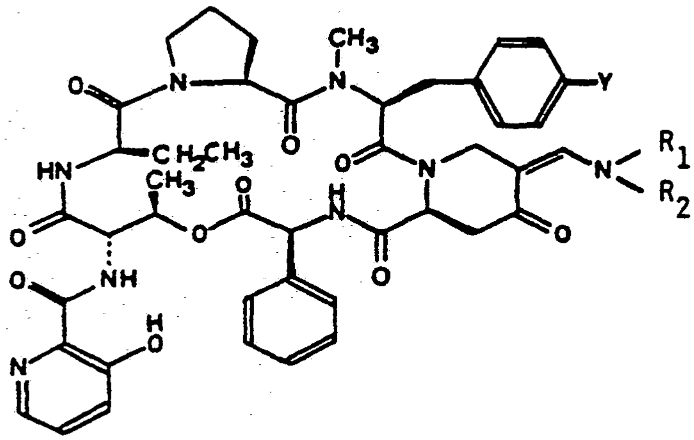

(I')

suivie éventuellement:

1) - soit de l'action d'un borohydrure alcalin en présence d'un acide organique fort pour obtenir un produit de formule générale (I) dans laquelle R représente un atome d'hydrogène,

2) - soit d'une transènamination par action d'une amine de formule générale:

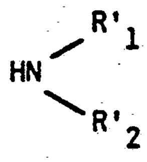

(V)

[dans laquelle $R'_1$ et $R'_2$ ont la définition donnée précédemment pour $R_1$ et $R_2$ à l'exception des significations définies pour la formule générale (III)] pour obtenir un produit de formule générale (I) dans laquelle Y est défini comme précédemment et R représente un radical de formule générale (II) dans laquelle $R_1$ et $R_2$ sont définis comme $R'_1$ et $R'_2$ ci-dessus,

3) - soit d'une hydrolyse pour obtenir un produit de formule générale:

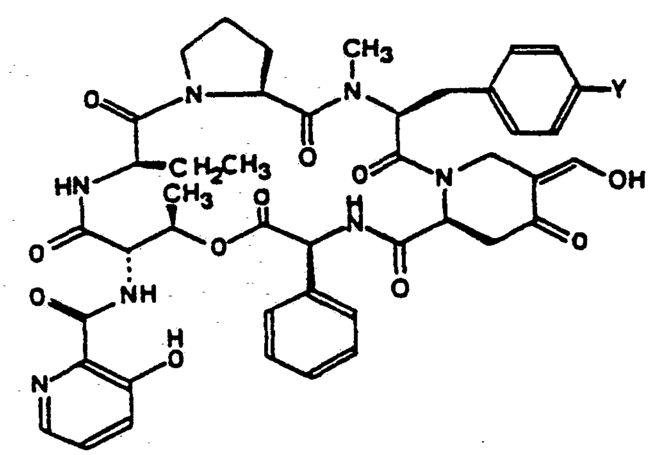

(VI)

puis éventuellement de la transformation en un produit de formule générale (I) pour lequel R est défini comme précédemment en c), par action d'un agent d'halogénation ou d'un produit de formule générale

R'X (VII)

dans laquelle R' est un radical triméthylsilyloxy, dialcoylphosphoryloxy ou un radical $-OSO_2R_3$ ou $-OCOR_4$ et

X représente un atome d'halogène.

Lorsque l'on prépare un produit de formule générale (I') au moyen d'un réactif de formule générale (III) dans laquelle $X_1$ et/ou $X_2$ représentent un radical amino substitué, il est préférable de choisir $X_1$ et/ou $X_2$ de telle manière que le radical amino substitué soit identique au groupement $-NR_1R_2$ présent sur la molécule. On utilise avantageusement le t.butoxy bis(diméthylamino)méthane.

Dans la pratique, la réaction du produit de formule générale (III) sur le produit de formule générale (IV) s'effectue généralement dans un solvant organique tel qu'un solvant chloré (par exemple le dichloro-1,2 éthane), ou un amide (par exemple le diméthylformamide) à une température comprise entre 0 et 25°C, de préférence à une température voisine de 20°C.

La réaction d'un borohydrure alcalin sur le produit de formule générale (I') s'effectue généralement au moyen d'un borohydrure ou d'un cyanoborohydrure de sodium dans un solvant organique tel qu'un éther (par exemple le tétrahydrofuranne) ou un alcool (par examplе l'isopropanol) en présence d'un acide organique fort tel que l'acide trifluoroacétique, à une température comprise entre 0°C et la température de reflux du mélange réactionnel On opère de préférence à une température voisine de 20°C.

La réaction de l'amine de formule générale (V) sur le produit de formule générale (I') s'effectue généralement dans un solvant organique tel que l'acide acétique ou un alcool (par exemple l'éthanol) à une température comprise entre 0 et 25°C, de préférence à une température voisine de 20°C.

L'hydrolyse du produit de formule générale (I') pour obtenir le produit de formule générale (VI) s'effectue généralement dans un acide dilué à une température voisine de 20°C. On utilise avantageusement une solution 0,1N d'acide chlorhydrique.

Lorsque l'on prépare un produit de formule générale (I) dans laquelle R est un atome d'halogène, par action d'un agent d'halogénation sur un dérivé de synergistine de formule générale (VI), on utilise avantageusement un dérivé halogéné du phosphore, notamment les composés d'addition d'halogène et de triarylphosphite, ou bien

- le dichlorotriphénylphosphorane ou le catéchyltrichlorophosphorane lorsque R est un atome de chlore ou
- le catéchyltribromophosphorane lorsque R est un atome de brome.

La réaction s'effectue généralement dans un solvant chloré tel que le chlorure de méthylène à une température comprise entre -20 et +20°C.

Lorsque l'on fait agir un produit de formule générale (VII) sur un dérivé de synergistine de formule générale (VI), on utilise de préférence un produit dans lequel X est un atome de chlore ou de brome.

La réaction s'effectue généralement dans un solvant organique tel que le chlorure de méthylène en présence d'un accepteur d'acide tel qu'une base organique comme la triéthylamine ou une base minérale comme un carbonate ou un bicarbonate alcalin, par exemple le bicarbonate de sodium ou de potassium. On opère généralement à une température comprise entre -20 et +20°C.

Les produits de formule générale (III) peuvent être préparés selon les méthodes décrites par H. Bredereck et coll., Chem. Ber. 101, 41 (1968), Chem. Ber. 101, 3958 (1968) et Chem. Ber. 106, 3725 (1973)

Les produits de formule générale (IV) sont des produits connus: la pristinamycine $I_A$ lorsque Y représente le radical diméthylamino, et le virginiamycine S lorsque Y représente un atome d'hydrogène.

Les nouveaux produits de formule générale (I) sont utiles notamment comme produits intermédiaires pour la préparation de dérivés de synergistines thérapeutiquement actives, de formule générale:

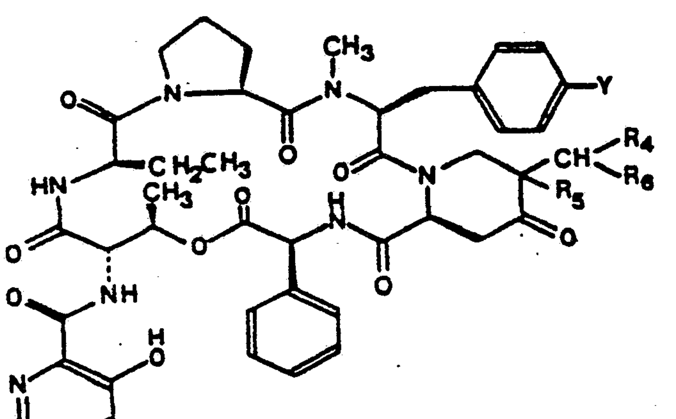

(VIII)

dans laquelle Y représente un atome d'hydrogène ou un radical diméthylamino et

A) soit $R_5$ et $R_6$ représentent chacun un atome d'hydrogène et $R_4$ représente un radical pyrrolidinyl-3 thio ou pipéridyl-3 ou 4 thio (ces radicaux étant éventuellement substitués par un radical alcoyle) ou bien $R_4$ représente

un radical alcoythio au substitué par un ou deux radicaux hydroxyaulfonyle, alcoylamimo, dialcoylamino ou par un ou deux cycles choisis parmi pipérazimo (éventuellement substitué par un radical alcoyle), morpholino, thiomorpholino, pipéridino, pyrrolidinyle-1, pipéridyl-2, 3 ou 4 et pyrrolidinyle-2 ou 3 (ces deux derniers cycles étant éventuellement substitués sur l'atome d'azote par un radical alcoyle).

B) soit $R_5$ et $R_6$ forment ensemble une liaison de valence et $R_4$ représente un radical pyrrolidinyl-3 amino, pipéridyl-3 ou 4 amino, pyrrolidinyl-3 oxy, pipéridyl-3 ou 4 oxy, pyrrolidinyl-3 thio, pipéridyl-3 ou 4 thio (ces radicaux étant éventuellement substitués sur l'atome d'azote du, cycle par un radical alcoyle), ou bien $R_4$ représente un radical alcoylamino, alcoyloxy ou alcoylthio substitués par un ou deux radicaux hydroxysulfonyle alcoylamino, dialcoylamino, trialcoylammonio ou imidazolyle-4 ou. 5 ou par un ou deux cycles choisis parmi pipérazino (éventuellement substitué par un radical alcoyle), morpholino, thiomorpholino, pipéridino, pyrrolidinyle-1, pipéridale-2, 3 ou 4 et pyrrolidinyle-2 ou 3 (ces deux derniers cycles étant éventuellement substitués sur l'atoms d'azote par un radical alcoyle), étant entendu que, dans la formuls générale (VIII) les radicaux alcoyle et portions alcoyle contiennent, 1 à 5 atomes de carbone et sont en chaîns droite ou ramifiée.

Pour obtenir les produits de formule générale (VIII) thérapeutiquement actifs, les produits selon l'invention peuvent être mis en oeuvre de la façon suivante:

- Pour préparer un produit de formule générale (VIII) dans laquelle les symboles sont définis comme précédemment en A), on fait réagir un produit de formule générale:

$R'_4$ - H (IX)

dans laquelle $R'_4$ a la définition donnée précédemment en A) pour $R_4$, sur un produit de formule générale (I) dans laquelle Y est défini comme précédemment et R représente un atome d'hydrogène.

On opère généralement dans un solvant organique tel qu'un alcool comme le méthanol ou un solvant chloré comme le chloroforme ou un mélange de ces solvants, à une température comprise entre 0°C et la température de reflux du mélange réactionnel, de préférence à une température de 20°C.

- Pour préparer un produit de formule générale (VIII) dans laquelle les symboles sont définis comme précédemment en B) à l'exception pour $R_4$ de représenter un radical pyrrolidinyl-3 oxy, pipéridyl-3 (ou -4) oxy ou alcoyloxy [(éventuellement substitués comme défini en B)], on fait réagir un produit de formule genérale:

$R''_4$ - H (X)

dans laquelle $R''_4$ a la définition donnée précédemment en B) pour $R_4$ à l'exception de représenter un radical pyrrolidinyl-3 oxy, pipéridyl-3 (ou -4) oxy ou alcolyoxy [éventuellement substitué comme défini en B)], sur un produit de formule générale (I) dans laquelle R est défini comme précédemment en b).

On opère généralement en milieu acide (par exemple acide acétique), avec ou sans solvant, à une température comprise entre 0 et 50°C, de préférence à une température voisine de 20°C, éventuellement en présence de quantités catalytiques d'acide trifluoracétique.

Le cas échéant le solvant peut être choisi parmi les éthers (tétrahydrofuranne), les alcools (éthanol) ou les solvants chlorés (chlorure de méthylène ou chloroforme par exemple).

- Pour préparer un produit de formule générale (VIII) dans laquelle les symboles sont définis comme précédemment en B), on fait réagir un produit de formule générale:

$R'''_4$ - H (XI)

dans laquelle $R'''_4$ a la définition donnée précédemment en B) pour $R_4$, sur un produit de formule générale (I) dans laquelle R est défini comme précédemment en c).

On opère généralement dans un solvant organique tel qu'un éther comme le tétrahydrofuranne ou un alcool comme l'éthanol ou un solvant chloré (chlorure de méthylène ou chloroforme par exemple) à une température voisine de 20°C, en présence d'une base telle qu'un hydrure alcalin ou un alcoolate alcalin comme l'éthylete de sodium ou le t.butylate de potassium. Lorsque $R'''_4$ est autre qu'un radical alcoyloxy substitué ou hétérocyclyloxy, il est également possible d'opérer soit en milieu neutre à une température comprise entre 0 et 50°C dans l'un des solvants cités ci-dessus, soit en milieu acide dans des conditions identiques à celles décrites précédemment pour l'action d'un produit de formule générale (XVI) sur un produit de formule générale (XIV).

Pour la préparation des composés thérapeutiquement actifs de formule générale (VIII) il est entendu pour l'homme du métier que, lorsque les différents radicaux de formules générales (IX), (X) ou (XI) renferment une fonction amine secondaire pouvant interférer avec la réaction, celle-ci doit être protégée avant de faire réagir les produits de formule générale (I), au moyen d'un radical protecteur que l'on élimine ensuite. On utilise a cet effet tout moyen de blocage habituellement employé pour protéger une fonction amine secondaire et pouvant être éliminé par la suite, sans toucher au reste de la molécule. Il est particulièrement avantageux d'utiliser comme radical protecteur le radical trifluoroacétyle. Celui-ci peut ensuite être éliminé à l'aide d'une solution aqueuse de bicarbonate alcalin tel que le bicarbonate de sodium ou de potassium.

Il est bien connu que les synergistines obtenues par fermentation constituent des produits très recherchés par les médecins pour le traitement de beaucoup d'affections dues à des bactéries Gram-positives (du genre Staphylocoques, Streptocoques, pneumocoques, entérocoques) et Gram-négatives (du genre Haemophilus, gonocoques, méningocoques). Toutefois, ces produits présentent l'inconvénient d'être insolubles en milieu aqueux et ne peuvent donc être administrés que par voie orale, généralement sous forme de gélules, de dragées ou de comprimés. Compte tenu de cette insolubilité, il est impossible d'utiliser les synergistines connues jusqu'ici lorsque le malade n'est pas en l'état de déglutir; c'est notamment le cas en pédiatrie et en réanimation, alors que le spectre d'activité de ces produits en ferait une indication précieuse dans un grand nombre de circonstances, par exemple dans les cas de septicémies comateuses.

Les produits de formule générale (VIII) présentent l'avantage considérable de pouvoir être solubilisés dans l'eau, à l'état de sels, aux doses thérapeutiquement utilisables, tout en conservant le spectre général d'activité des synergistines.

Ils sont notamment actifs in vitro sur Staphylococcus aureus Smith à des concentrations comprises entre 0,1 et 125 µg/ml.

Leur toxicité est généralement faible. Leur $DL_{50}$ est généralement supérieure à 300 mg/kg chez la souris par voie sous cutanée.

Les nouveaux produits de formule générale (I) ainsi que les produits thérapeutiquement actifs de formule générale (VIII) peuvent être purifiés par les méthodes habituelles telles que cristallisation, chromatographie et, le cas échéant, extractions successives en milieu acide et basique. Pour l'homme du métier connaissant la sensibilité des synergistines en milieu alcalin, il est évident qu'on entend par "milieu basique" un milieu juste suffisamment alcalin pour libérer la substance-mère de son sel d'addition avec un acide, c'est-à-dire un milieu dont le pH n'excède pas 7,5 à 8,

Lorsque les nouveaux produits de formule générale (I) se présentent sous des formes isomères, celles-ci peuvent être séparées par exemple par chromatographie liquide hautes performances.

Les nouveaux produits de formule générale (I) peuvent également être transformés, le cas échéant, en sels d'addition avec les acides par action d'un acide dans un solvant organique tel qu'un alcool, une cétone, un ester ou un solvant chloré. Le sel précipite, éventuellement après concentration de sa solution; il est séparé par filtration ou décantation. Les sels d'addition avec les acides peuvent également être obtenus à l'état de solutions aqueuses par addition d'une solution aqueuse d'acide correspondant sur le produit de formule générale (I).

En outre, les produits de formule générale (I) dans laquelle R représente un radical contenant une fonction carboxy, peuvent être transformés en sels métalliques ou en sels d'addition avec les bases azotées, de manière analogue à celle décrite précédemment pour les sels d'addition avec les acides mais en remplaçant l'acide par un hydroxyde métallique ou une base azotée.

Comme exemples de sels peuvent être cités les sels d'addition avec les acides minéraux tels que chlorhydrates, bromhydrates, iodhydrates, sulfates, nitrates phosphates ou organiques tels que acétates, propionates, succinates, maléates, fumarates, méthanesulfonates, p.toluènesulfonates, iséthionates, trifluoracétates, oxalates ou des dérivés de substitution de ces composés, et les sels avec les métaux alcalins ou alcalinoterreux tels que potassium, sodium, lithium, magnésium, ainsi que les sels d'addition avec les bases organiques azotées tels que diméthylamine, diéthylamine, diisopropylamine, dicyclohexylamine, N-éthylpipéridine, N-méthylmorpholine et éthanolamine.

D'un intérêt particulier sont les produits de formule générale (I) dans laquelle Y est défini comme précédemment et R représente soit un atome d'hydrogène ou un radical hydroxy, soit un radical $-NR_1R_2$ pour lequel $R_1$ et $R_2$ représentent un atome d'hydrogène ou un radical phényle éventuellement substitué par un radical dialcoylamino, ou alcoyle éventuellement substitué [par un radical hydroxy, mercapto, carboxy, pyridyle, anilino, alcoylamino ou dialcoylamino dont las parties alcoyle sont substituées par des radicaux hydroxy] ou un radical alcynyle contenant 3 ou 4 atomes de carbone.

Et parmi ces produits plus spécialement les produits suivants:

- diméthylaminométhyléne-5δ pristinamycine $I_A$
- diméthylaminométhylène-5δ virginiamycine S
- méthylène-5δ priatinamycine $I_A$
- hydroxyméthylène-5δ priatinamycine $I_A$
- méthylène-5δ virginiamycine S

Par ailleurs sont également intéressants les produits suivants:

- trifluoroacétoxyméthylène-5δ prisatinamycine $I_A$
- trifluoroacétoxyméthylène-5δ virginiamycine S

Les exemples suivants, donnés à titre non limitatif, montrent comment l'invention peut être mise en pratique. Les exemples d'application donnés également à titre non limitatif, montrent comment on peut mettre en oeuvre les produits de l'invention pour les transformer en produit de formule générale (VIII) thérapeutiquement actifs. Les spectres de RMN des produits illustrés dans les exemples et les exemples d'application présentent des caractéristiques générales qui sont communes à tous les produits et des caractéristiques particulières qui sont propres à chacun des produits en fonction des substituants variables dans les formules générales (I) et (VIII). Dans l'exemple 1, il est donné l'attribution de tous les protons de la molécule; dans les exemples et exemples d'application suivants ne sont mentionnés que les caractéristiques particulières dues aux radicaux variables. Tous les protons sont désignés selon la numérotation indiquée dans la formule de base suivante, recommandée par J.O. ANTEUNIS et coll., [Eur. J. Biochem., 58, 259 (1975)].

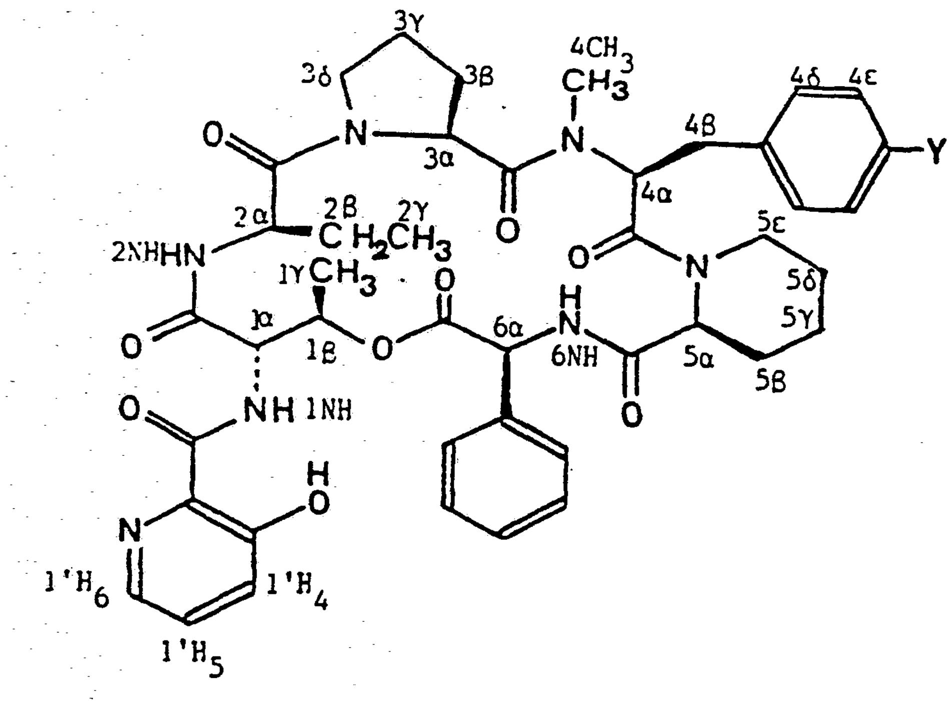

(XII)

Tous les spectres ont été faits à 250 MHz dans le deutérochloroforme; les déplacements chimiques sont exprimés en p.p.m. par rapport au signal du tétraméthylsilane. Les abréviations utilisées dans la suite sont les suivantes:

s = singulet
d = doublet
t = triplet
mt = multiplet
m = massif
dd = doublet de doublet
dt = doublet de triplet
ddd = doublet de doublet de doublet
dddd = doublet de doublet de doublet de doublet

Dans les exemples 2 à 24 ainsi que dans les exemples d'application sont donnés entre parenthèses, respectivement: le déplacement chimique, la forme du signal, l'intégration (nombre de protons, avec éventuellement le pourcentage d'isoméres) et l'attribution des protons.

Dans les exemples et exemples d'application qui suivent, on appelle chromatographie "flash" une technique de purification caractérisée en ce qu'on utilise une colonne de chromatographie courte et qu'on opère sous une pression moyenne (50 kPa) en utilisant une silice de granulométrie 40-63 µm, selon W.C. STILL, M. KAHN et A. MITRA [J. Org. Chem., 43, 2923 (1978)).

**Exemple 1**

A une solution de 46 g de pristinamycine $I_A$ dans 460 $cm^3$ de dichloro-1,2 éthane, on ajoute 230 $cm^3$ de tert-butoxy bis (diméthylamino)méthane; la solution obtenue est agitée pendant 18 heures à une température voisine de 20°C. Le mélange réactionnel est dilué par 1 litre de chlorure de méthylène puis lavé 3 fois par 3 litres au total d'une solution aqueuse à 0,4% de chlorure d'ammonium. La phase organique est séchée sur du sulfate de magnésium, filtrée, puis concentrée à sec sous pression réduite (2,7 kPa) à 30°C. Le résidu obtenu est trituré avec 600 $cm^3$ d'eau distillée; le mélange est filtré et le filtrat est séché et concentré à sec sous pression réduite (2,7 kPa) à 20°C. On obtient 41 g de diméthylaminométhylène-5δ pristinamycine $I_A$ brute sous forme d'une poudre beige. Ce produit est d'une qualité suffisante pour être utilisé tel quel dans les synthèses ultérieures. Il peut toutefois être purifié de la manière suivante:

23,5 g de diméthylaninométhyléne-5δ pristinamycine $I_A$ brute sont chromatographiés par chronatographie "flash" éluant: chloroforme - méthanol [98-2 en volumes]. Les fractions 16 à 25 sont réunies et concentrées à sec sous pression réduite (2,7 kPa) à 30°C.

On obtient ainsi 12 g de diméthylamino-5δ pristinamycine $I_A$ sous forme d'une poudre blanche fondant vers 195°C,

| δ (ppm) | Forme | Attribution |
|---|---|---|
| 11,6 | s | OH |
| 8,65 | d | 6NH |
| 8,40 | d | 1NH |
| 7,75 | dd | 1'$H_6$ |
| 7,40 | s | =CH-N< |
| 7,35 à 720 | m | 6γ + 6δ + 6ε + 1'$H_4$ + 1'$H_5$ |
| 6,95 | dd ) système AB | 4ε + 4δ |
| 6,55 | d ) | |
| 6,55 | m | 2NH |
| 5,85 | m | 1β |
| 5,77 | d | 6α |
| 5,50 | d | 5$ε_1$ |
| 5,25 | dd | 4α |
| 5,17 | d | 5α |
| 4,85 | dd | 1α |
| 4,80 | m | 2α |
| 4,55 | dd | 3α |
| 3,70 | d | 5$ε_2$ |
| 3,50 et 3,30 | m | 3$δ_1$ + 3$δ_2$ |
| 3,2 | s | 4N$CH_3$ |
| 3,10 | s | =CH-N($\underline{CH_3}$)$_2$ |
| 2,94 | s | 4N$(CH_3)_2$ |
| 2,94 | m | 4β |
| 2,50 | d | 5$β_1$ |
| 2,05 | m | 3$β_1$ |
| 1,60 | m | 2$β_1$+ 2$β_2$+ 3$γ_1$ |
| 1,25 | d | 1γ |
| 1,22 | m | 3$β_2$ + 3$γ_2$ |
| 1 | dd | 5$β_2$ |
| 0,9 | t | 2γ |

**Exemple 2**

En opérant d'une manière analogue à celle décrite à l'exemple 1, mais à partir de 2 g de virginiamycine S, et 10 $cm^3$ bis (diméthylamino) tert-butoxy méthane et après purification par chromatographie flash (éluant: chloroforme - méthanol (98-2 en volumes)] et concentration à sec des fractions 9 à 12 sous pression réduite (2,7 kPa) à 30°C, on obtient 0,8 g de diméthylamino-méthylène-5δ virginiamycine S sous forme d'une poudre jaune fondant vers 175°C.

Spectre RMN:
0,9 (m; 4H: $2\gamma + 5\beta_2$)
3,05 (s; 6H: $=CH\text{-}N(CH_3)_2$)
3,65 (d; 1H: $5\varepsilon_2$)
4,85 (d; 1H: $5\varepsilon_1$)
5,15 (dd; 1H: $5\alpha$)
7,10 à 7,40 (m; aromatiques +
= CH N<)
7,70 (dd; 1H : $1'H_6$)

**Exemple 3**

A une solution de 1,84 g de diméthylaminonéthyléne-5δ pristinamycine $I_A$ dans 20 $cm^3$ d'acide acétique, on ajoute lentement 10 $cm^3$ d'une solution éthanolique 4N d'ammoniac gazeux. La solution obtenue est agitée pendant 20 heures à une température voisine de 20°C, puis est versée lentement dans 200 $cm^3$ d'une solution aqueuse saturée de bicarbonate de sodiun. La suspension obtenue est extraite 3 fois par 300 $cm^3$ au total de chlorure de méthylène; les phases organiques sont réunies, séchées sur du sulfate de magnésium, filtrées et concentrées à sec sous pression réduite (2,7 kPa) à 30°C, Le résidu obtenu est purifié par chronatographie "flash" [éluant: chloroforme - méthanol (92-8 en volumes)]. Par concentration à sec des fractions 11 à 13 sous pression réduite (2,7 kPa) à 30°C, on obtient 1,3 g d'amino-méthyléne-5δ pristinamycine $I_A$ sous forme d'une poudre beige fondant vers 190°C,

Spectre RMN:
0,7 à 1,10 (mt, 4H: $2\gamma + 5\beta_2$)
7,15 à 7,53 (mt, 9H (dont 1 échangeable): $6\gamma + 6\delta + 6\varepsilon$ + 1H du $NH_2$ + $= C\underline{H}\text{-}NH_2 + 1'H_4 + 1'H_5$)
9,12 (s large, 1H (échangeable); 1H du $NH_2$).

**Exemple 4**

En opérant d'une manière analogue à celle décrite à l'exemple 3, mais à partir de 1,84 g de diméthylaminométhylène-5δ, pristinamycine $I_A$ et 1,9 $cm^3$ d'une solution éthanolique 7N de méthylamine et après purification par chromatographie "flash" [(éluant: chloroforme -méthanol (98-2 en volunes)]) et concentration à sec des fractions 16 et 17 sous pression réduite (2,7 kPa) à 30°C,on obtient 0,9 g de méthylaminométhylène-5δ pristinamycine $I_A$ sous forme d'une poudre jaune fondant vers 180°C,

Spectre RMN:
0,98 (dd, 1H: $5\beta_2$)
3,01 (d, 3H: $\text{-}NHC\underline{H}_3$)
7,17 à 7,42 (m, 8H: $6\gamma + 6\delta + 6\varepsilon + 1'H_5 + 1'H_4 + =C\underline{H}\,NHCH_3$)
9,80 (mt, 1H (échangeable): $N\underline{H}CH_3$)

**Exemple 5**

En opérant d'une manière analogue à celle décrite à l'exemple 3, mais à partir de 1,84 g de diméthylaminométhylène-5δ pristinamycine $I_A$ et 3,1 g de décylamune et après purification par chromatographie "flash" [éluant: chloroforme-méthanol (98-2 en volumes)] et concentration à sec des fractions 6 et 7 sous pression réduite (2,7 kPa) à 30°C, on obtient 1,3 g de décylaminonéthy-léne-5δ pristinamycine $I_A$ sous forme d'une poudre beige fondant vers 120°C.

Spectre RMH:
0,8 à 1,0 (mt; 7H: $2\gamma + 5\beta_2 + \text{-}(CR_2)_9\,C\underline{H}_3$)
1,10 à 1,45 (m; 21H: $1\gamma + 3\beta_2 + 3\gamma_2 + \text{-}CH_2(C\underline{H}_2)_8\,CH_3$)
3,10 à 3,45 (m; 7H: $4NCH_3 + 3\delta_2 + 4\beta_1 + =CH\text{-}NH\text{-}C\underline{H}_2\,(CH_2)_8\text{-}$)
7,20 à 7,50 (m; 8H: $6\gamma + 6\delta + 6\varepsilon + 1'H_5 + 1_4 > + \text{-}C\underline{H}\text{-}NH\text{-}$)

9,95 (m;, 1H (échangeable); =CH-N$\underline{H}$-)

**Exemple 6**

En oprérant d'une manière analogue à celle décrite à l'exemple 3, mais à partir de 1,84 g de diméthylaminonéthyléne-5δ pristinamycine $I_A$ et 1,42 g de pyrrolidine et après purification par chromatographie "flash" [éluant: chloroformemnéthanol (98-2 en volunes)] et concentration à sec des fractione 12 à 22 sous pression réduite (2,7 kPa) à 30° C, on obtient 0,8 g de (pyrrolidinyl-1 méthyléne)-5δ pristinamycine $I_A$ sous forme d'une poudre jaune fondant vers 260° C.

Spectre RMH:

0,9 (mt; 4H: 2γ + $5\beta_2$)

1,48 à 2,08 (m; 8H: $2\beta_7$ + $2\beta_2$ + $3\beta_1$ +

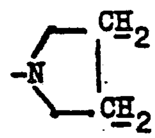

)

3,4 à 3,79 (m; 6H $3\delta_1$ + $5\varepsilon_2$ +

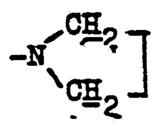

)

7,67 (s; 1H: =C$\underline{H}$

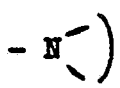

**Exemple 7**

En opérant d'une manière analogue à celle décrite à l'exemple 3, mais à partir de 1,84 g de diméthylaminométhylène-5δ pristinamycine $I_A$ et 1,4 g de N-méthyl propargylamine et après purification par chromatographie "flash" [éluant: acétate d'éthyle-métanol (90-10 en volumes)] et concentration à sec des fractions 9 à 21 sous pression réduite (2,7 kPa) à 30°C, on obtient 0,7 g de (N-méthyl propargylamino) méthylène-5δ pristinamycine $I_A$ sous forme d'une poudre jaune fondant ver 175° C.

Spectre RMN:

0,72 )dd; 1H: $5\beta_2$)

2,32 à 2,58 (mt + t; 2H: $5\beta_1$ + -C≡C-$\underline{H}$)

3,05 à 3,37 (m; 8H: -NC$\underline{H}_3$ + 4-NCH$_3$ + $3\gamma_2$ + $4\beta_1$)

4 (mt; 2H:

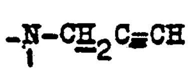

)

7,06 à 7,54 (m; 8H: 6γ + 6δ + 6ε, =C$\underline{H}$

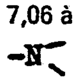

+ 1'$H_4$ + 1'$H_5$

**Exemple 8**

En opérant d'une manière analogue à celle décrite à l'exemple 3, mais à partir de 1,84 g de diméthylaminométhylène-5δ pristinamycine $I_A$ et 0,36 g d'aniline et après purification par chromatographie "flash" [éluant: acétate d'éthyle] et concentration à sec des fractions 4 à 10 sous pression réduite (2,7 kPa) à 30°C, on obtione 1,6 g de phénylaminométhylène-5δ pristinamycine $I_A$ fontant vers 270°C (avec décomposition).

Spectre RMN:

0,95 (dd; 1H: $5\beta_2$)

2,45 (d; 1H: $5\beta_1$)

3,60 (d; 1H: $5\varepsilon_2$)
5,05 (d; 1H:$5\varepsilon_1$)
5,20 (d; 1H: $5\alpha$)
7,25 (d; 1H: -N$\underline{H}$-$C_6H_5$)
7 à 7,50 (m; 13H: $6\gamma + 6\delta + 6\varepsilon + 1'H_4 + 1'H_5$ -NH—$C_6\underline{H}_5$ + =C$\underline{H}$-NH-)
7,75 (dd; 1H: $1'H_6$

**Exemple 9**

En opérant d'une manière analogue à celle décrite à l'exemple 3, mais à partir de 0.90 g de diméthylaminométhylène-5δ pristinamycine $I_A$ et 0.22 g de N-méthyl aniline et après purification par chromatographie "flash" (éluant: acétate d'éthyle-méthanol (90-10 en volumes)] et concentration à sec des fractions 10 à 14 sous pression réduite (2.7 kPa) à 30°C. on obtient 0,4 g de (N-méthyl phénylamino)méthylène-5δ pristinamycine $I_A$ sous forme d'une poudre jaune fondant vers 170°C.

Spectre RMN:
0,90 (m; 4H: $5\beta_2 + 2\gamma$)
2,50 (d; 1H: $5\beta_2$)
3,50 (s; 3H:-

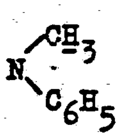
)

5,15 (m; 2H: $4\alpha + 5\alpha$)
7,10 à 7,40 (m; 13H: $6\gamma + 6\delta + 6\varepsilon + 1'H_4 + 1'H_5$-

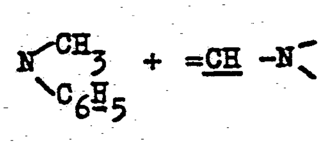
+ )

7,75 (dd; 1H: $1'H_6$)

**Exemple 10**

En opérant d'une manière analogue à celle décrite à l'exemple 3, mais à partir de 1,84 g de diméthylaminométhylène-5δ pristinamycine $I_A$ et 1,36 g de diméthylamino-4 aniline et après purification par chromatographie "flash" [éluant: chloroforme-méthanol (98-2 en volumes)] et concentration à sec des fractions 3 à 5 sons pression réduite (2,7 kPa) à 30°C. on obtient 1 g de (diméthylamino-4 phényl) aminométhylène-5δ pristinamycine $I_A$ sous forme d'une poudre brune fondant vers 165°C.

Spectre RMN:
0,90 (m; 4H: $2\gamma + 5\beta_2$)
2,40 (d; 1H: $5\beta_1$)
2,95 (s; 6H:

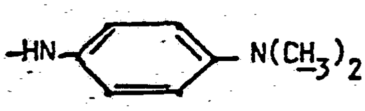
)

4,55 (d; 1H: $5\varepsilon_2$)
5,05 (d; 1H: $5\varepsilon_1$)
5,15 (m; 2H: $4\alpha + 5\alpha$)
7 (m; 4H:

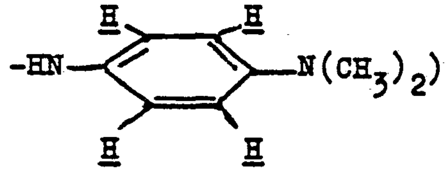

7,30 (dans massif: =CH-NH-)
7,80 (dd; 1H: $1'H_6$)

**Exemple 11**

En opérant d'une manière analogue à celle décrite à l'exemple 3, mais à partir de 2,77 g de diméthylaminométhylène-5δ pristinamycine $I_A$ et 3,54 g de N-(hydroxy-2 éthyl) propylènediamine et après purification par chromatographie "flash" [éluant: chloroforme-méthanol (85-15 en volumes)] et concentration à sec des fractions 19 à 25 sous pression réduite (2,7 kPa) à 30° C, on obtient 0,95 g de [(hydroxy-2 éthylamino)-3 propyl] aminométhylène-5δ pristinamycine $I_A$ sous forme d'une poudre jaune fondant vers 162° C.

Spectre RMN:
0.90 (1H sous 2γ: $5\beta_2$)
2,45 (d, 1H: $5\beta_1$)
2,80 (m; 2H: =CH-NH-$CH_2$-)
3,15 (m; 4H: -$CH_2$-NH-$CH_2$-)
3,50 (large d; 1H: $5\varepsilon_1$)
3,75 (large m; 1H: -$CH_2$-OH)
5,15 (large s; 1H: 5α)
6,60 (m; 1H: -$CH_2$NH-$CH_2$-)
7,20 à 7,50 (m aromatique + =-CHNH-)
7,80 (dd; 1H: $1'H_6$)
10,00 (large m; 1H: =CH-NH-)

**Exemple 12**

En opérant d'une manière analogue à celle décrite à l'exemple 3, mais à partir de 2,76 g de diméthylaminométhylène-5δ pristinamycine $I_A$ et 4,86 g de N-(amino-3 propyl)diéthanolamine et après purification par chronatographie "flash" [éluant chloroforme-méthanol (85-15 en volumes)] et concentration à sec des fractions 13 à 19 sous pression réduite (2,7 kPa) à 30° C, on obtient 0,95 g de [N-bis(hydroxy-2 éthyl)amino-3 propyl] aninométhylène-5δ pristinamycine $I_A$ sous forme d'une poudre jaune fondant vers 164° C.

Spectre RMN:
0,90 (sons le triplet de 2γ, 1H : $5ß_2$)
2,50 (d; 1H: $5\beta_1$)
2,60 (m; 4H: 2 fois-

FIG21/10
-$CH_2$- de la chaîne)
3,10 (m; 4H: 2 fois N-$CH_2$- de la chaîne)
3,45 (large d; 1H: $5\varepsilon_2$)
3,65 (large m; 4H: 2 fois -$CH_2$OH)
4,90 (large d; 1H: $4\varepsilon_1$)
5,15 (large d; 1H: 5α)
7,75 (dd; 1H: 1,$H_6$)
10,25 (m; 1H: =CH-NH-)

**Exemple 13**

En opérant d'une manière analogue à celle décrite à l'exemple 3, mais à partir de 1,84 g de diméthylaminométhylène-5δ pristinamycine $I_A$ et 1,54 g d'amino-2 éthanethiol et après purification par

chromatographie "flash" [éluant: acétate d'éthyleméthanol (90-10 en volumes)] et concentration à sec des fractions 8 à 14 sous pression réduite (2,7 kPa) à 30° C, on obtient 0,6 g de (mercapto-2 éthylamino)méthylène-5δ pristinamycine $I_A$ sous forme d'une poudre jaune fondant vers 160° C.

Spectre RMN:
0.90 (m; 4H: 2γ + $5\beta_2$)
1,45 (t; 1H: -SH)
2,45 (d; 1H: $5\beta_1$)
2,70 (m; 2H: $-CH_2S-$)
3,40 (m; 2H: $-NH-CH_2-$)
3,50 (d; 1H: $5\varepsilon_2$)
4,95 (dd; 1H: $5\varepsilon_1$)
7,15 à 7,50 (m; 8H: 6γ + 6δ + 6ε + $1'H_1$-$1'H_5$ =CH-NH-)
7,80 (dd; 1H: $1'H_6$)
10 (m; 1H: =CH-NH-)

**Exemple 14**

En opérant d'une manière analogue à celle décrite à l'exemple 3, mais à partir de 2,76 g de diméthylaminométhylène-5δ pristinamycine $I_A$ et 4,08 g de N-phényléthylènediamine et après purification par chromatographie "flash" [éluant chloroforme-méthanol (98-2 en volumes)] et concentration à sec des fractions 14 à 24 sous pression réduite (2,7 kPa) à 30°C, on obtient 1,2 g de (phénylamino-2 éthyl)aminométhylène-5δ pristinamycine $I_A$ sous forme d'une poudre jaune fondant vers 172° C.

Spectre RMN:
0,95 (sous le méthyle 2γ, 1H: $5\beta_2$)
2,45 (d; 1H: $5\beta_1$)
3,10-3,40 (m; 4H: $-CH_2-CH_2NHC_6H_5$)
3,45 (d; 1H: $5\varepsilon_2$)
5,15 (large d; 1H: 5α)
6,55 à 6,80 (m; 4H: -nH

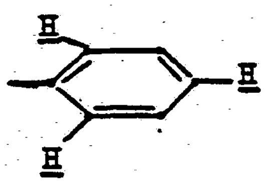 )

7,25 à 7,50 (m; sans les aromatiques: =CH-)
7,80 (dd; 1H: $1'H_6$
10,00 (m; 1H: =CH-NH-)

**Exemple 15**

En opérant d'une manière analogue à celle décrite à l'exemple 3, mais à partir de 1,84 g de diméthylaminométhylène-5δ pristinamycine $I_A$ et 2,44 g d'(amino-2 éthyl)-2 pyridine et après purification par chromatographie "flash' [éluant chloroformeméthanol (95-5 en volumes)] et concentration à sec des fractions 9 à 12 sous pression réduite (2,7 kPa) à 30°C, on obtient 1,2 g de [(pyridyl-2)-2 éthyl] aminométhylène-5δ pristinamycine $I_A$ sous forme d'une poudre jaune fondant vers 170° C.

Spectre RMN:
0,90 (sous t de 2γ, 1H: $5\beta_2$)
2,45 (d; 1H: $5\beta_1$)
3,25 (m; 2H: $-CH_2-NH-$)
3,45 (m; 3H: dont $5\varepsilon_2$)
4,85 (m; 3H: dont $5\varepsilon_1$)
5,10 (d; 1H: 5α)
7,10 à 7,45 (m; aromatiques dont 3 H:

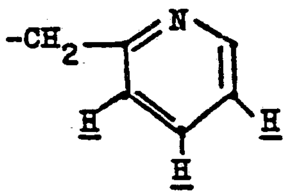

+ 1H sous aromatiques : =CH-)

7,75 (dd; 1H: 1'$H_6$)
8,55 (large d; 1H:

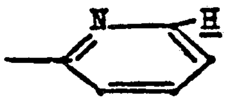

)

10,00 (m; 1H: N<u>H</u>-$CH_2$-)

**Exemple 16**

En opérant d'une manière analogue à celle décrite à l'exemple 5, mais à partir de 1,84 g de diméthylaminométhylène-5δ pristinamycine $I_A$ et 1,5 g de glycine et après purification par chromatographie "flash' [éluant: chlorure de méthylène-méthanol (80-20 en volumes)] et concentration à sec des fractions 9 à 16 sous pression réduite (2,7 kPa) à 30° C, on obtient 0,7 g de carboxyméthyl-aminométhylène-5δ pristinamycine $I_A$ sous forme d'une poudre ocre fondant à une température supérieure à 270° C.

Le spectre infrarouge présente les bandes caractéristiques des pristinamycines: 3300 $cm^{-1}$, 1745 $cm^{-1}$, 1660 - 1620 $cm^{-1}$ 1525 $cm^{-1}$, 810 $cm^{-1}$. 700 $cm^{-1}$ ainsi que celles du groupe $-CO^-_2$ (sous forme d'un sel interne): 1660 à 1620 $cm^{-1}$ et 1410 $cm^{-1}$.

**Exemple 17**

A une solution de 12 g de diméthylaminométhylène-5δ pristinamycine $I_A$ dans 230 $cm^3$ de tétrahydrofuramine contenant 1,2 $cm^3$ d'acide trifluoroacétique, on ajoute 0,43 g de cyanoborohydrure de sodium; la solution obtenue est agitée pendant 4 heures à une température voisine de 20° C, puis est concentrée à sec sous pression réduite (2,7 kPa) à 30° C. Le résidu obtenu est purifié par chromatographie "flash" [éluant: chloroforme-méthanol (95-5 em volumes)]; les fractions 4 à 15 sont concentrées à sec sons pression réduite (2,7 kPa) à 30° C. On obtient ainsi 5,5 g de méthylène-5δ pristinamycine $I_A$ sous forme de cristaux blancs fondant à 245° C.

Spectre RMN:
0,55 (dd; 1H: 5$\beta_2$)
2,40 (d; 1H: 5$\beta_1$)
3,55 (dd; 1H: 5$\varepsilon_2$)
5,25 (m; 2H: 5α + 5$\varepsilon_1$)
5,30 et 6,10 (2 s; 2H:

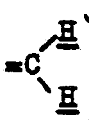

)

7,85 (dd; 1H: 1'$H_6$)

La diméthylaminométhylène-5δ pristinamycine $I_A$ peut être préparée comme décrit à l'exemple 1.

**Exemple 18**

A une solution de 19,5 g de diméthylaminométhylène-5δ pristinamycine $I_A$ dans 570 $cm^3$ d'isopropanol, refroidie à 5°C, on ajoute en 15 minutes environ 20 $cm^3$ d'acide trifluoroacétique. On ajoute ensuite 0,4 g de borohydrure de sodium, puis agite 20 heures à une température voisine de 20°C. Le mélange réactionnel est ensuite concentré à sec sous pression réduite (2,7 kPa) à 30°C; le résidu obtenu est dissous dans 400 $cm^3$ de chlorure de méthylène. La solution est lavée par 2 fois 200 $cm^3$ d'eau distillée, séchée sur du sulfate de sodium, filtrée et concentrée à sec sous pression réduite (2,7 kPa) à 30°C. Le résidu est purifié par chromatographie "flash" [éluant: chlorure de méthylène-méthenol (97/3 en volumes)]; en concentrant à sec les fractions 17 à 34 sous pression réduite (2,7 kPa) à 30°C, on obtient 5,7 g de méthylène-5δ pristinamycine $I_A$ sous forme d'une poudre jaune fondant vers 230°C (produit brut d'une pureté suffisante pour être mis en oeuvre tel quel dens les étapes ultérieures).

**Exemple 19**

On opère d'une manière analogue à celle décrite à l'exemple 17 pour la méthylène-5δ pristinamycine $I_A$, mais à partir de 2 g de diméthylaminométhylène-5δ virginiamycine S et 74 mg de cyanoborohydrure de sodium. Après purification par chromatographie "flash" [éluant: chloroforme-méthanol (98-2 en volumes)] et concentration à sec des fractions 2 à 5 sous pression réduite (2,7 kPa) à 30°C, on obtient 1 g de méthylène-5δ virginiamycine S sous forme d'une poudre beige fondant vers 190°C.

Spectre RMN:
0,35 (dd, 1H: $5\beta_2$)
2,45 (dd; 1H: $5\beta_1$)
3,55 (dd, 1H: $5\varepsilon_2$)
5,25 (dd. 1H: $5\varepsilon_1$)
5,25 (m, 1H: $5\alpha$)
5,30 et 6,15 (2s, 2H: 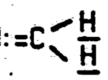 )

7,75 (dd, 1H: $1'H_6$)
La diméthylaminométhylène-5δ virginiamycine S peut être préparée comme décrit à 1 exemple 2.

**Exemple 20**

A 420 $cm^3$ d'une solution aqueuse 0,1 N d'acide chlorhydrique, on ajoute sous agitation 10,6 g de diméthylaminométhylène-5δ pristinamycine $I_A$. La solution obtenue est ensuite agitée pendant 3 heures à une température voisine de 20°C. On ajoute alors goutte à goutte 30 $cm^3$ d'une solution aqueuse saturée de bicarbonate de sodium de manière à obtenir un pH voisin de 4. Le produit qui précipite est séparé par filtration puis lavé 3 fois par 30 $cm^3$ au total d'eau distillée. Après séchage sous pression réduite (2,7 kPa) à une température voisine de 20°C, on obtient 9,5 g d'hydroxyméthylène-5δ pristinamycine $I_A$ sous forme d'une poudre beige. Ce produit est d'une qualité suffisante pour être utilisé tel quel dans les phases ultérieures. Il peut toutefois être purifié de la manière suivante:

9,5 g d'hydroxyméthylène-5δ pristinamycine $I_A$ brute sont dissous dans 50 $cm^3$ d'acétate d'éthyle et la solution obtenue est versée sur 100 g de gel de silice contenus dens une colonne de 2,8 cm de diamètre. On élue d'abord avec 400 $cm^3$ d'acétate d'éthyle; l'éluat correspondant est éliminé. On élue ensuite avec 1600 $cm^3$ d acétate d'éthyle; l'éluat correspondant est concentré à sec sous pression réduite (2,7 kPa) à 30°C. On obtient ainsi 6,3 g d'hydroxyméthylène-5δ pristinamycine $I_A$ sous forme de cristaux blancs fondant à 220°C.

Spectre RMN:
0,69 (dd; 1H: $5\beta_2$)
2,43 (d; 1H: $5\beta_1$)
3,40 (d; 1H: $5\varepsilon_2$)
4,0 à 4,2 (m; 3H: $4\alpha + 5\varepsilon_1 + 5\alpha$)
8,15 (s; 1H: =C<u>H</u>-OH)
11,63 (s large; 1H: =CH-O<u>H</u>)

**Exemple 21**

A une solution de 1,8 g d'hydroxyméthylène-5δ pristinamycine $I_A$ dans 20 $cm^3$ de chlorure de méthylène contenant 0,2 g de triéthylamine, on ajoute à une température voisine de -20°C 0,14 $cm^3$ de chlorure d'acétyle

puis on laisse remonter la température à environ 20° C.

Le mélange réactionmel est agité ensuite pendant 20 heures à cette température, puis concentré à sec sous pression réduite (2,7 kPa) à 30° C; le résidu obtenu est purifié par chromatographie "flash" [éluant: acétate d'éthyle]. Après concentration à sec des fractions 4 à 7 sous pressiom réduite (2,7 kPa) à 30° C, on obtient 0,7 g d'acétoxyméthylène-5δ pristinamycine $I_A$ sous forme d'une poudre jaune fondant vers 160° C.

Spectre RMN:
0,60 (dd; 1H: $5\beta_2$)
2,25 (s; 3H: -CO-C$\underline{H}_3$)
2,45 (d; 1H: $5\beta_1$)
3,45 (dd; 1H: $5\varepsilon_2$)
5,25 (dd; 1H: $5\alpha$)
5,45 (d; 1H: $5\varepsilon_1$)
7,10 à 7,45 (m; 8H: $6\gamma + 6\delta + 6\varepsilon + 1'H_4 + 1'H_5 +$ =C$\underline{H}$-O-)
7,85 (dd; 1H: $1'H_6$)

**Exemple 22**

En opérant d'une manière analogue à celle decrite l'exemple 21, mais à partir de 1,8 g d'hydroxyméthylène-5δ pristinamycine $I_A$ et 0,34 g de chlorophosphate de diéthyle et après purification par chromatographie "flash" [éluant: acétate d'éthyle-méthanol (90-10 en volumes)] et concentration à sec des fractions 6 à 14 sous pression réduite (2,7 kPa) à 30° C, on obtient 0,8 g de diéthoxyphosphoryloxyméthylène-5δ pristinamycine $I_A$ sous forme d'une poudre jaune fondant vers 150° C.

Spectre RMN:
0,55 (dd; 1H - $5\beta_2$)
1,30 (td; 6H: -PO(O-$CH_2$-C$\underline{H}_3$)$_2$)
2,40 (d; 1H: $5\beta_1$)
3,40 (dd; 1H: $5\varepsilon_2$)
4,25 (ddd; 4H: -PO(O-C$\underline{H}_2$-$CH_3$)$_2$
5,25 (d; 1H: $5\alpha$)
5,40 (d; 1H: $5\varepsilon_1$)
7,10 à 7,55 (m; 8H: $6\gamma + 6\delta + 6\varepsilon +$ =C$\underline{H}$-O- + $1'H_5 + 1'H_4$)
7,85 (dd; 1H x 0,85: $1'H_6$ 1er isomère)
8 (dd; 1H x 0,15: $1'H_6$ 2eme isomère)

**Exemple 23**

A une solution de 2,7 g d'hydroxyméthylène-5δ pristinamycine $I_A$ dans 30 $cm^3$ de chlorure de méthylène on ajoute à une température voisine de -30° C 0,42 $cm^3$ de triéthylamine puis 0,57 g de chlorure de l'acide p-toluènesulfonique et on laisee remonter la température à environ 20° C.

Le mélange réactionnel est agité ensuite pendant deux heures à cette température, puis concentré à sec sous pression réduite (2,7 kPa) à 30° C; le résidu obtenu est purifié par chromatographie "flash" [éluant: chlorure de méthylène-méthanol (96-4 en volumes)]. Après concentration à sec des fractions 4 à 6 sous pression réduite (2,7 kPa) à 30° C, on obtient 2,2 g de (méthyl-4 phényl)sulfonyloxyméthylène-5δ pristinamycine $I_A$ sous forme d'une poudre blanche fondant vers 265° C.

Spectre RMN:
0,50 (dd; 1H: $5\beta_2$)
2,35 (s; 3H:

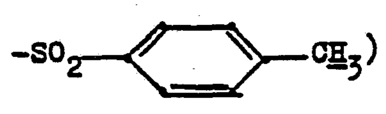

3,30 (dd; 1H: $5\varepsilon_2$)
5,25 (d; 1H: $5\alpha$)
5,30 (dd; 1H: $5\varepsilon_1$
7,35 à 7,90 (système AB + m, 8 H: $4\delta + 4\varepsilon +$

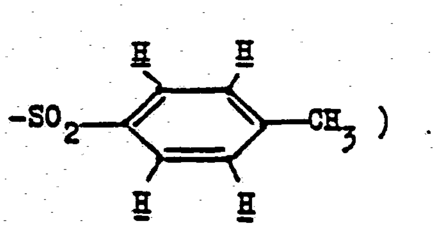

7,85 (dd; 1H: 1'$H_6$

**Exemple 24**

Dans une solution de 1,3 $cm^3$ de triphénylphosphite dans 25 $cm^3$ de chlorure de méthylène, on fait passer un courant de chlore gazeux jusqu'à obtenir une couleur jaune-vert persistante, en maintenant la température entre -20°C et -15°C. On ajoute ensuite 6 gouttes de triphénylphosphite pour décolorer la solution, puis 4,1 g d'hydroxyméthylène-5δ pristinamycine $I_A$, toujours en maintenant la température entre -20°C et -15°C. On agite la solution obtenue pendant 1 heure à -15°C, puis on ajoute goutte à goutte une solution de 0,4 $cm^3$ de pyridine dans 25 $cm^3$ de chlorure de méthylène. On agite ensuite le mélange réactionnel 30 minutes à une température voisine de 20°C, puis on ajoute 0,46 $cm^3$ d'acide chlorhydrique concentré (d = 1,19) et 50 $cm^3$ de chlorure de méthylène. Le mélange est lavé 4 fois par 100 $cm^3$ au total d'eau distillée; la phase organique est séchée sur du sulfate de magnésium, filtrée puis concentrée à sec sous pression réduite (2,7 kPa) à 30°C. Le résidu obtenu est purifié par chromatographie "flash" [éluant: acétate d'éthyle]; après concentration à sec des fractions 7 à 9 sous pression réduite (2,7 kPa) à 30°C, on obtient 1,2 g de chloromé-thylène-5δ pristinamycine $I_A$ sous forme d'une poudre beige fondant vers 190°C.

Spectre RMN:
0,55 (dd; 1H: 5$\beta_2$)
2,45 (d; 1H: 5$\beta_1$)
3,45 (dd; 1H: 5$\varepsilon_2$)
5,30 (d; 1H: 5α)
5,45 (d; 1H: 5$\varepsilon_1$)
7,15 à 7,60 (m; 8 H: 6γ + 6δ + 6ε + 1'$H_4$ + 1'$H_5$ + =C$\underline{H}$-Cl)
7,85 (dd; 1H: 1'$H_6$)

**Exemple d'application 1**

A une solution de 5,5 g de diméthylaminométhylàne-5δ pristinamycine $I_A$ dans 60 $cm^3$ d'acide acétique on ajoute goutte à goutte 5,3 g de diméthylamino-2 éthylamine de manière à ne pas dépasser 25°C La solution obtenue est agitée pendant 20 heures à une température voisine de 20°C, puis est versée lentement dans une solution aqueuse saturée de bicarbonate de sodium, le mélange obtenu est extrait 2 fois par 750 $cm^3$ au total de chlorure de méthylène. Les phases organiques sont réunies, séchées sur du sulfate de magnésium, filtrées et concentrées à sec sous pression réduite (2,7 kPa) à 30°C. Le résidu est purifié par chromatographie "flash" [éluant: chloroforme-méthanol (90-10 en volumes)]; les fractions 10 à 12 sont concentrées à sec sous pression réduite (2,7 kPa) à 30°C. On obtient ainsi 3 g de (diméthylamino-2 éthyl) aminométhy-lène-5δ pristinamycine $I_A$ sous forme d'une poudre beige fondant vers 180°C.

Spectre RMN:
0,90 (mt, 4H: 2γ + 5$\beta_2$)
2,25 (mt, 6H: -N($CH_3$)$_2$)
2,50 (mt, 3H: -$CH_2$N
<
\
+ 5$\beta_1$)
3,25 (mt, 2H: -N-C$\underline{H_2}$-)
3,50 (mt, 2H: 5$\varepsilon_2$ + 3$\delta_1$)
4,90 (mt, 1: 5$\varepsilon_1$)
entre 7,15 et 7,4 (m, 1H;

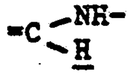 )

9,90 (mt, 1H (échangeable $D_2O$): -N$\underline{H}$-)

On obtient une solution aqueuse à 1 % de (diméthylamino-2 éthyl) aminoéthylène-5δ pristinamycine $I_A$ (produit AG), avec:

produit AG ......................0,1 g
eau distillée ...............qsp 10 cm³

**Exemple d'application 2**

En opérant d'une manière analogue à celle décrite à l'exemple d'application 1, mais à partir de 1,84 g de diméthylamino-méthylène-5δ pristinamycine $I_A$ et 2,8 cm³ de diéthylamino-2 éthylamine et après purification par chromatographie "flash" [éluant: chloroformeméthanol (96-4 en volumes)] et concentration à sec des fractions 9 à 13 sous pression réduite (2,7 kPa) à 30°C, on obtient 1 g de (diéthyl-amino-2 éthyl) aminométhylène-5δ pritinamycine $I_A$ sous forme d'une poudre jaune fondant vers 150°C.

Spectre RMN
0,9 (mt, 4H: 2γ + 5$\beta_2$)
1,1 (mt, 6H: -N($CH_2$-$C_{H3}$)$_2$)
2,45 (d, 1H: 5$\beta_1$)
3,1 à 3,4 (m, 6H: -C$\underline{H}_2$

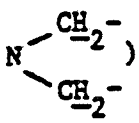 )

3,50 (mt, 2H: 5$\varepsilon_2$ + 3$\delta_1$)
4,90 (m, 1H: 5$\varepsilon_1$)
9,9 (m, 1H (échangeable: =CH-N$\underline{H}$-)

On obtient une solution aqueuse à 5 % de (diéthylamino-2 éthyl) aminométhylène-5δ pristinamycine $I_A$ (produit AH), à l'état de chlorhydrate, avec:

produit AH .......................0,1 g
acide chlorhydrique 0,1N .........1 cm³
eau distillée ......................qsp 2 cm³

**Exemple d'application 3**

En opérant d'une manière analogue à celle décrite à l'exemple d'application 1, mais à partir de 2,76 g de diméthylamino-éthylène-5δ pristinamycine $I_A$ et 2,22 g de N-méthyléthylènediamine et après purification par chromatographie "flash" [éluant: chloroformeméthanol (95-5 en volumes)] et concentration à sec des fractions 16 à 20 sous pression réduite (2,7 kPa) à 30°C, on obtient 1,3 g de (méthylamino-2 éthyl)aminométhylène-5δ pristinamycine $I_A$ sous forme d'une poudre jaune fondant à 174°C.

Spectre RMN:
0,90 (m, 4H: 5$\beta_2$)
2,50 (m, 1H: 5$\beta_1$)
2,7-3,6 (m, 4H: -NH-(C$\underline{H}_2$)$_2$NH-)
3,0 (sous un massif, s, 3H: -NHC$\underline{H}_3$)
7,15-7,40 (m, 1H: =C$\underline{H}$NH-)
9,90 (m, 1H: = CH-N$\underline{H}$$CH_3$)

On obtient une solution aqueuse à 1 % de (méthylamino-2 éthyl)aminométhylène-5δ pristinamycine $I_A$ (produit AI), à l'état de chlorhydrate, avec:

produit AI ........................... 0,03 g
acide chlorhydrique 0,1N ............ 0,31 cm³
eau distillée .................. qsp 3 cm³

**Exemple d'application 4**

En opérant d'une manière analogue à celle décrite à l'exemple d'application 1, mais à partir de 1,84 g de diméthylamino-méthylène-5δ pristinamycine $I_A$ et 2,5 $cm^3$ de diméthylamino-3 propylamine et après purification par chromatographie "flash [éluant: chloroforme-méthanol (90-10 en volumes)] et concentration à sec des fractions 12 à 15 sous pression réduite (2,7 kPa) à 30°C on obtient 0,7 g de (diméthylamino-3 propyl)aminométhylène-5δ pristinamycine $I_A$ sous forme d'une poudre jaune fondant vers 155°C.

Spectre RMN:
0,80 à 1,05 (mt, 4H: $2\gamma + 5\beta_2$)
1,80 (mt, 2H: $-CH_2C\underline{H_2}-CH_2-$)
2,40 (s, 6H x 0,15: $-\underline{N(CH_3)_2}$ 2ème isomère)
2,40 à 2,60 (mt, 3H: $5\beta_1$ +
$-CH_2-N<$ )
3,30 (mt, 2H: $-NH-C\underline{H_2}-$)
3,50 (mt, 2H: $3\delta_1 + 5\varepsilon_2$)
4,90 (mt, 1H: $5\varepsilon_1$)
9,65 (mt, 1H x 1H x 0,15: =CH-N$\underline{H}$-2ème isomère)
9,90 (m, 1H x 0,85: =CH-N$\underline{H}$-ler isomère)

On obtient une solution à 6,6 % de (diméthylamino-3 propyl)aminométhylène-5δ pristinamycine $I_A$ (produit AJ), à l'état de chlorhydrate, avec:
produit AJ ........................... 0,1 g
acide chlorhydrique 0,2N ........... .. 0,51 $cm^3$
eau distillée .....................qsp 1,5 $cm^3$

**Exemple d'application 5**

En opérant d'une manière analogue à celle décrite à l'exemple d'application 1, mais à partir de 2,76 g de diméthylaminométhy-lène-5δ pristinamycine $I_A$ et 3,06 g de diméthylamino-1 propylamine-2 et après purification par chromatographie flash [éluant: chloroforme-méthanol (99-1 en volumes)] et concentration à sec des fractions 11 à 22 sous pression réduite (2,7 kPa) à-30°C, on obtient 1,0 g de (diméthylamino-3 propyl-2) aminométhylène-5δ pristinamycine $I_A$ sous forme d'une poudre jaune fondant vers 160°C.

Spectre RMN:
1,05 (d, 3H:
$-CH-C\underline{H_3}$ )
2,30 (s, 6H: $-CH_2-N(C\underline{H_3})_2$)
2,45 (d, 1H: $5\beta_1$)
2,80 (m, 1H:
$-\underline{CH}CH_3$ )
3,30 (sous un massif: $-NH-C\underline{H_2}-$)
3,45 (m, 2H: $5\varepsilon_2 + 3\delta_1$)
4,90 (m, 1H: $5\varepsilon_1$)
7,15-7,40 (m, 1H: $-C\underline{H}NH-$)
9,90 (m, 1H: $-CH-N\underline{H}-$)

On obtient une solution aqueuse à 1 % de (diméthylamino-3 propyl-2) aminométhylène-5δ pristinamycine $I_A$ (produit AK), à l'état de chlorhydrate, avec:
produit AK ........................... 20 mg
acide chlorhydrique 0,1N ............. 0,2 $cm^3$
eau distillée ..................... qsp 2 $cm^3$

**Exemple d'application 6**

En opérant d'une manière analogue à celle décrite à l'exemple d'application 1, mais à partir de 2,76 g de diméthylamino-méthylène-5δ pristinamycine $I_A$ et 1,53 g de diméthylamino-2 propylamine et après purification par chromatographie "flash" [éluant: chloroforme-méthanol (95-5 en volumes)] et concentration à sec des fractions 10 à 14 sous pression réduite (2,7 kPa) à 30°C, on obtient 0,85 g de (diméthylamino-2 propyl) aminométhylène-5δ pristinamycine $I_A$ sous forme d une poudre orange fondant vers 175°C.

Spectre RMN:

0,90 (m, 4H - 2γ $5\beta_2$)
1,05 (d, 3H:
$>CH-C\underline{H}_3$)
2,30 (s, 6H: $-CH(CH_3)N(C\underline{H}_3)_2$)
2,45 (d, 1H: $5\beta_1$)
2,80 (m, 1H: $-C\underline{H}CH_3$)
3,30 (sous un massif, 2H: $-NH-C\underline{H}_2-$)
3,45 (m, 2H: $5\varepsilon_2 + 3\delta_1$)
4,90 (m, 1H: $5\varepsilon_1$)
7,15-7,40 (m, IH: $=C\underline{H}NH-$)
9,90 (m, 1H: $=CH-N\underline{H}-$)

On obtient une solution aqueuse à 10 % de (diméthyl-amino-2 propyl) aminométhylène-5δ pristinamycine $I_A$ (produit AL), à l'état de chlorhydrate, avec

produit AL .................... 0,03 g
acide chlorhydrique 0,1N ....... 0,31 $cm^3$

**Exemple d'application 7**

En opérant d'une manière analogue à celle décrite à l'exemple d'application 1, mais à partir de 1,84 g de diméthyleminométhylè-ne-5δ pristinamycine $I_A$ et 3,16 g d'amino-2 diéthylamino-5 pentane et après purification par chromatographie "flash" [éluant: chloroformeméthanol (90-10 en volumes)] et concentration à sec des fractions 15 à 27 sous pression réduite (2,7 kPa) à 30°C, on obtient 0,9 g de (diéthylamino-5 pentyl-2) aninométhylène-5δ pristinamycine $I_A$ sous forme d une poudre beige fondant vers 160°C.

Spectre RMN:
1,00 (dd, 1H: $5\beta_2$)
1,25 (mt, gH: $-N(CH_2C\underline{H}_3)_2$)
2,45 (d, 1H: $5\beta_1$)
2,7-3,0 (m, 6H: $-C\underline{H}_2$

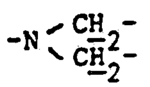 )

3,45 (dd, 1H: $5\varepsilon_2$)
7,30 (sous les aromatiques: $=C\underline{H}-NH-$)
7,85 (dd, 1H: $1'H_6$)
10 (m large, 1H:
$-N\underline{H}-CH-$)

On obtient une solution aqueuse à 1 % de (diéthylamino-5 pentyl-2) aminoéthylène-5δ pristinamycine $I_A$ (produit AM), à l'état de chlorhydrate, avec:

produit AM ..........................0,02 g
acide chlorhydrique 0,1N ...............0,2 $cm_3$
eau distillée .......................qsp 2 $cm_3$

**Exemple d'application 8**

En opérant d'une manière analogue à celle décrite à l'exemple d'application 1, mais à partir de 1,84 g de diméthylamino-méthyléne-5δ pristinamycine $I_A$ et 2,28 g de N-(amino-2 éthyl) pyrrolidine et après purification par chromatographie "flash" [éluant: chloroforme-méthanol (98-2 en volumes)] et concentration à sec des fractions 15 à 24 sous pression réduite (2,7 kpa) à 30°C, on obtient 0,95 g de (pyrrolidino-2 éthyl) aminométhyléne-5δ pristinamycine $I_A$ sous forme d'une poudre jaune fondant à 183°C.

Spectre RMN:
0,90 (mt, 4H: $2\gamma + 5\beta_2$)
1,80 (mt, 4H:

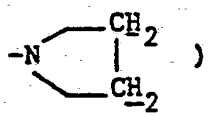

2,70 (mt, 6H: -C$\underline{H}_2$

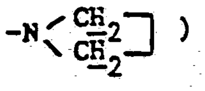

3,45 (m, 4H: -NH-C$\underline{H}_2$- + 5$\varepsilon_2$ + 3$\delta_1$)
4,90 (m, 1H: 5$\varepsilon_1$)
7,2-7,4 (m: Ar + 1'$H_4$ + 1'$H_5$ + =C$\underline{H}$-)
9,90 (mt, 1H: =CHN$\underline{H}$CH$_2$-)

On obtient une solution à 1 % de (pyrrolidino-2 éthyl) aminométhylène-5δ pristinamycine $I_A$ (produit AN), à l'état de chlorhydrate, avec

produit AN ..................... 0,02 g
acide chlorhydrique 0,1N ....... 0,2 cm$^3$
eau distillée .................qsp 2 cm$^3$

**Exemple d'application 9**

En opérant d'une manière analogue à celle décrite à l'exemple d'application 1, mais à partir de 2,76 g de diméthylamimométhylèn -5δ pristinamycine $I_A$ et 1,92 g de N-(amino-3 propyl)pyrrolidine et après purification par chromatographie "flash" [éluant: chloroformeméthanol (95-5 en volumes)] et concentration à sec des fractions 10 à 15 sous pression réduite (2,7 kPa) à 30°C, on obtient 1,25 g de (Pyrrolidino-3 propyl) aminométhylène-5δ pristinamycine $I_A$ sous forme d'une poudre jaune fondant à 170°C.

Spectre RMN:
0,95 (m, 1H: 5$\beta_2$)
1,95 (m, 7H:

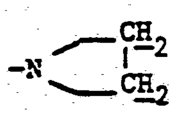

+ 3$\beta_1$ +

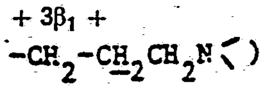

2,45 (d large, 1H: 5$\beta_1$)
2,80 (sous un massif, 6H: -C$\underline{H}$

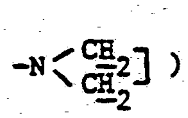

3,30 (mt, 2H: -NH-C$\underline{H}_2$-)
3,50 (mt, 2H: 3$\delta_1$ + 5$\varepsilon_2$)
4,90 (m, 1H: 5$\varepsilon_1$)
7,15-7,40 (m, 1H: =C$\underline{H}$NH-)
9,90 (mt, 1H: =CH-N$\underline{H}$-)

On obtient une solution aqueuse à la 1 % de (pyrrolidino-3 propyl) aminométhylène-5δ pristinamycine $I_A$ (produit AO), avec:

produit AO ...........................0,03 g
eau distillée .........................3 cm$^3$

**Exemple d'application 10**

En opérant d'une manière analogue à celle décrite à l'exemple d'application 1, mais à partir de 2,76 g de diméthylamino-5δ pristinamycine $I_A$ et 3,85 g de N-(amino-2 éthyl) pipéridine et après purification par chromatographie "flash" [éluant chloroforme-méthanol (99-1 en volumes)] et concentration à sec des fractions 13 à 17 sous pression réduite (2,7 kPa) à 30° C, on obtient 1,5 g de (pipéridino-2 éthyl) aminométhylène-5δ pristinamycine $I_A$ sous forme d une poudre jaune fondant vers 162° C.

Spectre RMN:

0,90 (m, 4H: $2\gamma + 5\beta_2$)

1,60 (mt, 6H:

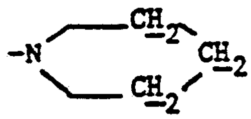 )

2,40 (m, 6H: $\underline{H}_2$C-

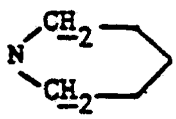 )

2,7-3,5 (m sous un massif, 2H: -NH-C$\underline{H}_2$-)

3,45 (mt, 2H: $3\delta_1 + 5\varepsilon_2$)

4,90 (mt, 1H: $5\varepsilon_1$)

7,15-7,40 (m1H: =C$\underline{H}$NH-)

9,90 (mt, 1H: =CH-N$\underline{H}$-)

On obtient une solution aqueuse à 1 % de (pipéridino-2 éthyl)aminométhylène-5δ pristinamycine $I_A$ (produit AP), à l'état de chlorhydrate, avec:

produit AP ........................0,02 g
acide chlorhydrique 0,1N...........0,2 $cm^3$
eau distillée ................qsp 2 $cm^3$

**Exemple d'application 11**

En opérant d'une manière analogue à celle décrite à l'exemple d'application 1, mais à partir de 2,6 g de N-(amino-2 éthyl) morpholine et 1,84 g de diméthylaminométhylène-5δ pristinamycine $I_A$ et après purification par chromatographie "flash" [éluant: chloroformeméthanol (95-5 en volumes)] et concentration à sec des fractions 21 à 30 sous pression réduite (2,7 kPa) à 30° C, on obtient 0,8 g de (morpholino-2 éthyl) aminométhylène-5δ pristinamycine $I_A$ sous forme d'une poudre beige fondant vers 172° C.

Spectre RMN:

0,90 (m, 1H: $5\beta_2$)

2,50 (m, 7H: $5\beta_1$ + -$C_{H2}$

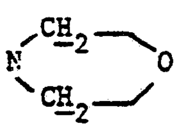 )

3,30 (m, 2h: -NH-C$\underline{H}_2$-)

3,50 (m, 2H: $5\varepsilon_2 + 3\delta_1$)

3,70 (mt, 4H:

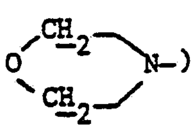

4,90 (m, 1H: =C$\underline{H}$

7,2-7,4 (m, 1H : =C$\underline{H}$

9,90 (mt, 1H: =CH-N$\underline{H}$-$CH_2$-)

On obtient une solution aqueuse à 1 % de (morpholino-2-éthyl) aminoméhylène-5δ pristinamycine $I_A$ (produit

AQ), à létat de chlorhydrate, avec:

produit AQ........................0,02 g
acide chlorhydrique 0,1N ..............0,2 $cm^3$
eau distillée ......................qsp 2 $cm^3$

**Exemple d'application 12**

En opérant d'une manière analogue à celle décrite à l'exemple d'application 1, mais à partir de 2,76 g de diméthylaminométhylè-ne-5δ pristinamycine $I_A$ et 3,66 g d'aminométhyl-2 éthyl-1 pyrrrolidine et après purification par chromatographie "flash" [éluant: chloroforme-méthanol (99-1 en volumes)] et concentration à sec des fractions 10 à 14 sous pression réduite (2,7 kPa) à 30°C, on obtient 1,3 g d (éthyl-1 pyrrolidinyl-2) méthylaminométhylàne-5δ pristinamycine $I_A$ sous forme d'une poudre beige fondant vers 160°C.

Spectre RMN:
1,10 (t, 3H: $-CH_2-C\underline{H}_3$)
1,60 (m, 4H:

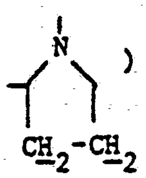

1,95 (m, 1H: =CH-NH-)
2,8-3,6 (m, 4H:

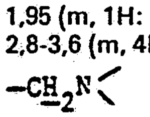

+ $-C\underline{H}_2NH-$)
7,15-7,40 (m, 1H: =C$\underline{H}$NH-)

On obtient une solution aqueuse à 1 % d (éthyl-1 pyrrolidinyl-2) méthylaminométhylène-5δ pristinamycine $I_A$ (produit AR), à 1 état de chlorhydrate, avec:

produit AR .......................... 0,02 g
acide chlorhydrique 0,1N ... ........ 0,2 $cm^3$
eau distillée ..................qsp 2 $cm^3$

**Exemple d'application 13**

En opérant d'une manière analogue à celle décrite à l'exemple d'application 1, mais à partir de 2,77 g de diméthylamino-méthylène-5δ pristinamycine $I_A$ et 3,4 g d'amino-3 méthyl-1 pipéridine et après purification par chromatographie "flash" [éluant: chloroformeméthanol (95-5 en volumes)] et concentration à sec des fractions 7 à 16 sous pression réduite (2,7 kPa) à 30°C, on obtient 0,8 g de (méthyl-1 pipéridyl-3) aninométhylène-5δ pristinamycine $I_A$ sous forme d'une poudre beige fondant à 177°C.

Spectre RMN:
0,90 (mt, 4H: $2\gamma + 5\beta_2$)
1,5-2,10 (mt, 7H: $2\beta_1 + 2\beta_2 + 3\gamma_1$ +

+ 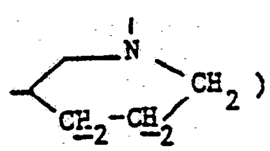

2,30 (s,3H:

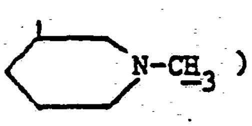

2,45 (d, 1H: $5\beta_1$)
2,65 (mt, 1H:

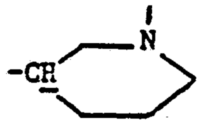 )

2,90 (mt, 4H: $4\beta_2$ +

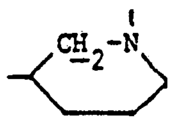 ou 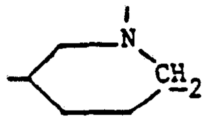 )

3,20 (mt, 7H: -NC$\underline{H}_3$ en 4 + $3\delta_2$ + $4\beta_1$ +

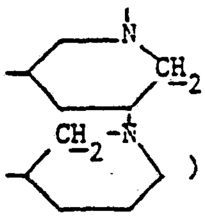

ou )

7,15-7,40 (m, 1H: =C$\underline{H}$NH-)
7,80 (mt, 1H: 1'$H_6$)
9,90 (mt, 1H: =CH-N$\underline{H}$-)
11,60 (s large, 1H: O$\underline{H}$)

On obtient une solution aqueuse à 1 % de (méthyl-1 pipéridyl-3) aminométhylène-5δ pristinamycine $I_A$ (produit AS), à l'état de chlorhydrate, avec

produit AS ........................... 0,02 g
acide chlorhydrique 0,1N ............ 0,2 cm³
eau distillée ...................qsp 2 cm³

L'amino-3 méthyl-1 pipéridine peut être préparée selon la méthode décrite par L.M. WERBEL, A. CURRY, E.F. ELSLAGER, C. HESS, J. Heterocyclic Chem. 10, 363 (1973).

**Exemple d'application 14**

En opérant d'une manière analogue à celle décrite à l'exemple d'application 1, mais à partir de 13,8 g de diméthylaminométhylè-ne-5δ pristinamycine $I_A$ et 3,4 g d'amino-4 méthyl-1 pipéridine et après purification par chromatographie "flash" [éluant: chloroformeméthanol (92,5-7,5 en volumes) et concentration à sec des fractions 15 à 20 sous pression réduite (2,7 kPa) à 30°C, on obtient 4,0 g de (méthyl-1 pipéridyl-4) aminométhylène-5δ pristinamycine $I_A$ sous forme d'une poudre jaune fondant à 208° C.

Spectre RMN:
0,40 (m, 4H: $2\gamma$ + $2\beta_2$)
2,0 (m, 4H:

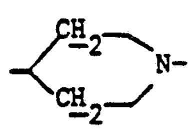 )

2,35 (s, 3H:

$>N-CH_3$)

2,45 (d, 1H: $5\beta_1$)
2,90

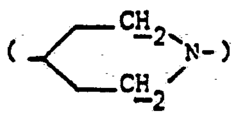

3,20 (sous un massif, 1H:

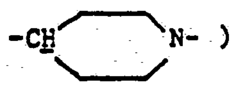

3,50 (d, 1H: $5\varepsilon_2$)
4,85 (sous un massif, 1H: $5\varepsilon_1$)
6,65 (d, 1H: =C<u>H</u>NH-)
9,70 (dd, 1H x 0,15: =CH-N<u>H</u>- Ier isomère)
10,03 (dd, 1H x 0,85: =CH-N<u>H</u>- 2ème isomère)

On obtient une solution aqueuse à 10 % de (méthyl-1 pipéridyl-4) aminométhylène-5δ pristinamycine $I_A$ (produit AT), à l'état de chlorhydrate, avec:

produit AT ........................ 0,03 g
acide chlorhydrique 0,1N ..............0,3 $cm^3$
eau distillée ...............qsp 0,3 $cm^3$

L'amino-4 méthyl-1 pipéridine peut être préparée par la méthode décrite par E.F. ELSLAGER, L.M. WERBEL, A. CURRY, N. HEADEN, J. JOHNSON, J. Med. Chem. 17, 99 (1974).

**Exemple d'application 15**

En opérant d'une manière analogue à celle décrite à l'exemple d'application 1, mais à partir de 0,8 g de diméthylaminométhylè-ne-5δ virginiamycine S et 1,02 g d'amino-4 méthyl-1 pipéridine et après purfication par chromatographie "flash" [éluant: chloroformeméthanol (90-10 en volumes)] et concentration à sec des fractions 3 à 7 sous pression réduite (2,7 kPa) à 30°C, on obtient 0,3 g de (méthyl-1 pipéridyl-4) aminométhylène-5δ virginiamycine S sous forme d'une poudre beige fondant vers 195°C.

Spectre RMN:
0,9 (m, 4H: $2\gamma + 5\beta_2$)
2,30 (s, 3H:

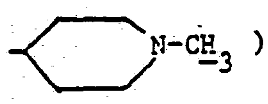

2,80 à 3,30 (m, 5H:

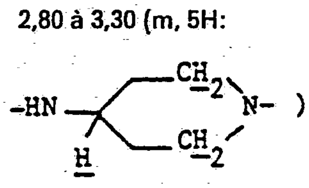

3,55 (dd, 1H: $5\varepsilon_2$)
4,90 (m, 1H: $5\varepsilon_1$)
7,10 à 7,40 (m: aromatiques + =C<u>H</u>-NH-)
7,70 (dd, 1H: $1H_6$)
10,1 (m, 1H: =CH-N<u>H</u>-)

On obtient une solution aqueuse à 5 % de (méthyl-1 pipéridyl-4) aminométhylène-5δ virginiamycine S (produit AU); à l'état de chlorhydrate, avec:

produit AU ...............0,1 g
acide chlorhydrique 0,1N............1,05 $cm_3$
eau distillée.................qsp 2 $cm^3$

**Exemple d'application 16**

En opérant d'une manière analogue à celle décrite à l'exemple d'application 1, mais à partir de 2,76 g de diméthylaminométhylè-ne-5δ pristinamycine $I_A$ et 2,15 g d'(amino-2 éthyl)-1 méthyl-4 pipérazine et après purfication par chromatographie "flash" [éluant: chloroforme-méthanol (95-5 en volumes)] et concentration à sec des fractions 10 à 16 sous pression réduite (2,7 kPa) à 30°C, on obtient 0,9 g de [(méthyl-4 pipérazinyl-l)-2 éthyl] aminométhylène-5δ pristinamycine $I_A$ sous forme d'une poudre jaune fondant à 150°C.

Spectre RMN:
1,00 (m, 1H - $5\beta_2$)
2,30 (s, 3H:
$>N-CH_3$)
2,50 (m, 9H: $-CH_2-$ pipérazine + $5\beta_1$)
,90 (sous un massif $-CH_2-CH_2$
$-N<$)
3,30 (m, 2H: $-NH-C\underline{H}_2-$)
3,50 (m, 2H: $5\varepsilon_2 + 3\delta_1$)
4,90 (m, 1H: $5\varepsilon_1$)
7,15-7,40 (m, 1H: $=C\underline{H}NH-$)
9,90 (m, 1H: $=CH-N\underline{H}-$)

On obtient une solution aqueuse à 10 % de [(méthyl-4 pipérazinyl-1)-2 éthyl] aminométhylène-5δ pristinamycine $I_A$ (produit AV), à l'état de chlorhydrate, avec:
produit AV ........................ 15 mg
acide chlorhydrique 0,1N ... ...... 0,15 $cm^3$

L'(amino-2 éthyl)-1 méthyl-4 pipérazine peut être préparée de la manière suivante:
A une solution de 10,0 g de bromhydrate de bromo-2 éthylamine dans 60 $cm^3$ d éthanol absolu, on ajoute 9,75 g de N-méthylpiperazine. La solution obtenue est agitée pendant 16 heures à une température voisine de 20°C puis l'éthanol est éliminé sous pression réduite (2,7 kPa) à 30°C. Le résidu huileux est repris par 50 $cm^3$ de chloroforme; le mélange obtenu est agité avec 20 $cm^3$ d'une solution aqueuse de soude ION. La phase aqueuse est extraite 3 fois avec 150 cm3 au total de chloroforme. Les phases organiques sont réunies, séchées sur du sulfate de sodium, filtrées et concentrées à sec sous pression réduite (2,7 kPa) à 30°C. Le résidu est distillé sous pression réduite (2,7 kPa); on obtient ainsi 4,5 g d'(amino-2 éthyl)-1 méthyl-4 pipérazine sous forme d'une huile jaune [PE (2,7 kPa) = 118-119°C].

**Exemple d'application17**

En opérant d'une manière analogue à celle décrite à l'exemple d'application 1, mais à partir de 4,0 g de diméthylaminométhylène-5δ pristinamycine $I_A$ et de 0,55 g d'histamine et après purification par chromatographie "flash' [éluant: chloroforme-méthanol (95-5 en volumes)] et concentration à sec des fractions 25 à 50 sous pression réduite (2,7 kPa) à 30°C, on obtient 2,04 g d'[(imidazo-lyl-4)-2 éthyl] aminométhylène-5δ pristinamycine $I_A$ sous forme d'une poudre jaune fondant à 138°C.

Spectre RMN:
0,90 (m, 4H: $2\gamma + 5\beta_2$)
2,40 (d large, 1H: $5\beta_1$)
2,90 (sous un massif, m, 1H: $5\varepsilon_1$)
3,50 (d, 4H: $5\varepsilon_{2>l} + 3\delta_1$ + $-NH-C\underline{H}_2-$)
4,80 (sous un massif, 1H: $5\varepsilon_1$)
6,65 (m, 2H:
$H_5$ + $>NH$ histamine)
7,50 (s, 1H, H en 2 de l'histamine)
entre 7,15 et 7,40 (m, 1H: $=C\underline{H}NH-$)
9,65 (m, 1H x 0,15: $=CH-N\underline{H}$ - 2ème isomère)
9,95 (m, 1H x 0,85: $=CH-N\underline{H}$- ler isomère)

On obtient une solution aqueuse à 10 % d'[(imidazolyl-4)-2 éthylaminomdthylène]-5δ pristinamycine $I_A$ (produit AW), à l'état de chlorhydrate, avec:
produit AW ... 0,1 g
acide chlorhydrique 0,1N .....qsp 1 $cm^3$

**Exemple d'application 18**

A une solution de 1,84 g de diméthylaminométhylène-5δ pristinamycine $I_A$ dans 40 $cm^3$ d acide acétique, on ajoute 2,1 g de diméthylamino-2 éthanethiol. La solution obtenue est agitée pendant 20 heures à une température voisine de 20°C, puis est versée lentement dans une solution aqueuse saturée de bicarbonate de sodium; le mélange obtenu est extrait 3 fois par 400 $cm^3$ au total de Chlorure de méthylène. Les phases organiques sont réunies, séchées sur du sulfate de magnésium, filtrées et concentrées à sec sous pression réduite (2,7 kPa) à 30°C. Le résidu obtenu est purifié par chromatographie "flash" [éluant chloroforme-méthanol (96-4 en volumes)]; les fractions 5 et 6 sont réunies et concentrées à sec sous pression réduite (2,7 kPa) à 30°C. On obtient ainsi 0,8 g de (diméthylamino-2 éthyl) thiométhylène-5δ pristinamycine $I_A$ sous forme d une poudre jaune fondant vers 150°C.

Spectre RMN:
0,68 (dd, 1H - 5$\beta_2$)
2,32 (s, 6H x 0,85: $-CH_2N(CH_3)_2$ Ier isomère)
2,35 (s, 6H x 0,15: $-CH_2N(CH_3)_2$ 2ème isomère)
2,45 (d, 1H: 5$\beta_1$)
2,65 (mt, 2H: $-S\underline{CH_2}-$)
3,05 (t, 2H:
$-\underline{CH_2}N<$)
3,43 (dd, 1H: 5$\varepsilon_2$)
5,15 (dans un massif: 5$\varepsilon_1$)
7,60 (s large, 1H: $=C\underline{H}S-$)
7,83 (mt, lH: 1'$H_6$ deux isomères)

On obtient une solution aqueuse à 1 % de (diméthylamino-2 éthyl) thiométhylène-5δ pristinamycine $I_A$ (produit AX), à l'état de chlorhydrate, avec
produit AX ......................... 0,1 g
acide chlorhydrique 0,1N ........... 1 $cm^3$
eau distillée ................. qsp 10 $cm^3$

**Exemple d'application 19**

En opérant d'une manière analogue à celle décrite à l'exemple d'application 18, mais à partir de 3,68 g de diméthylamino-méthylène-5δ pristinamycine $I_A$ et 8,5 g de diéthylamino-2 éthenethiol et après purification par chromatographie "flash" [éluant: chloroformeméthanol (96-4 en volumes)] et concentration à sec des fractions 13 à 20 sous presaion réduite (2,7 kPa) à 30°C, on obtient 0,85 g de (diéthyl-amino-2 éthyl) thiométhylène-5δ pristinamycine $I_A$ sous forme d'une poudre beige fondant vers 192°C.

Spectre RMN:
0,65 (dd, 1H: 5$\beta_2$)
1,05 (t, 6H: $-N(CH_2\underline{CH_3})_2$
2,42 (d, 1H: 5$\beta_1$)
2,60 (q, 4H: $-N(\underline{CH_2}CH_3)_2$)
3,42 (dd, 1H: 5$\varepsilon_2$)
5,10 (sous un massif, 1H: 5$\varepsilon_1$)
7,58 (s large 1H: $=C\underline{H}-S-$)
7,82 (dd, 1H x 0,95: 1'$H_6$ Ier isomère)
7,98 (dd, 1H x 0,05: 1'$H_6$ 2ème isomère)

On obtient une solution aqueuse à 1 % de (diéthylamino-2 éthyl) thiométhylène-5δ pristinamycine $I_A$ (produit AY), à l'état de chlorhydrate, avec:
produit AY ......................... 0,04 g
acide chlorhydrique 0,1N .......... 0,4 $cm^3$
eau distillée ................qsp 4 $cm^3$

**Exemple d'application 20**

En opérant d'une manière analogue à celle décrite à l'exemple d'application 18, mais à partir de 3 g de diméthylaminométhylène-5δ pristinamycine $I_A$ et 0,4 g de diméthylamino-3 propanethiol et après purification par chromatographie "flash" [éluant: chloroforme-méthanol (92,5-7,5 en volumes)] et concentration à sec des fractions 10 à 17 sous pression réduite (2,7 kPa) à 30°C, on obtient 0,85 g de (diméthylamino-3 propyl) thiométhyléne-5δ pristinamycine $I_A$ sous forme d'une poudre beige fondant vers 170°C.

Spectre RMN:

0,70 (dd, 1H: 5$\beta_2$)
1,90 (m, 2H: -S-$CH_2\underline{CH_2}$
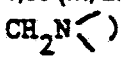
2,20 (s, 6H: -N($C\underline{H_3}$)$_2$)
2,40 (d, 1H: 5$\beta_1$)
2,90 (m, 2H: -$C\underline{H_2}$
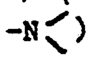
3,45 (dd, 1H: 5$\varepsilon_2$)
7,65 (s large, 1H: =CH-S-)

On obtient une solution aqueuse à 1 % de (diméthylamino-3 propyl) thiométhylène-5δ pristinamycine $I_A$ (produit AZ), à l'état de chlorhydrate, avec:

produit AY .......................... 0,03 g
acide chlorhydrique 0,1N . .......... 0,3 $cm^3$
eau distillée .................. qsp 3 $cm^3$.

**Exemple d'application 21**

En opérant d'une manière analogue à celle décrite à l'exemple d'application 18, mais à partir de 1,8 g de diméthylaminométhylè-ne-5δ virginiamycine S et 0,48 g de diméthylamino-3 propanethiol et après purification par chromatographie "flash" [éluant: chloroformeméthanol (95-5 en volumes)] et concentration à sec des fractions 5 à 14 sous pression réduite (2,7 kPa) à 30°C, on obtient 0,7 g de (diméthylamino-3 propyl) thiométhylène-5δ virginiamycine S sous forme d'une poudre beige fondant vers 140°C.

Spectre RMN:
0,50 (dd, 1H: 5$\beta_2$)
2 (m, 2H: -$SCH_2$-$C\underline{H_2}$-$CH_2$
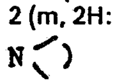
2,35 (s, 6H: -S($CH_2$)$_3$N($C\underline{H_3}$)$_2$)
2,60 (t, 2H: -$S\underline{CH_2}$-$CH_2CH_2$
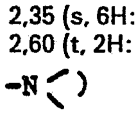
3 (t, 2H: -$SCH_2CH_2C\underline{H_2}$
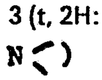
3,35 (dd, 1H: 5$\varepsilon_2$)
4,90 (dd, 1H: 5$\varepsilon_1$)
5,20 (m, 1H: 5α)
7,60 (s large, 1H: =$C\underline{H}$-S-)
7,80 (dd, 1H: 1'$H_6$)

On obtient une solution aqueuse à 10 % de (diméthylamino-3 propyl) thiomethylène-5δ virginiamycine S (produit AAA), à l'état de chlorhydrate, avec:

produit AAA ......................... 0,1 g
acide chlorhydrique 0,2N ............ 0,52 $cm^3$
eau distillée ................... qsp 1 $cm^3$

**Exemple d'application 22**

En opérant d'une manière analogue à celle décrite à l'exemple d'application 18, mais à partir de 4 g de diméthylaminométhylène-5δ pristinamycine $I_A$ et 0,7 g de diméthylamino-3 méthyl-2 propanethiol et après purification par chromatographie "flash" [éluant: chloroforme-méthanol (94-6 en volumes)] et concentration à sec sous pression réduite (2,7 kPa) à 30°C, on obtient 0,96 g de (diméthyl-amino-3 méthyl-2 propyl) thiométhylène-5δ pristinamycine $I_A$ sous forme d'une poudre beige fondant à 234°C.

Spectre RMN:
0,65 (dd, 1H: 5$\beta_2$)
1,05 (d, 3H:
-CHC$\underline{H_3}$)
2,25 (s, 6H: -N($C\underline{H_3}$)$_2$)
2,40 (d, 1H: 5$\beta_1$)

3,15 et 2,90 (système ABX, 2H: -C$\underline{H}_2$
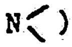)
3,45 (d large, 2H: 5ε)
7,75 (dd, 1H x 0,80: 1'$H_6$ ler isomère)
7,95 (dd, 1H x 0,20: 1'$H_6$ème isomère)

On obtient une solution aqueuse à 1 % de (diméthylamino-3 méthyl-2 propyl) thiométhylène-5δ pristinamycine $I_A$ (produit AAB), à l'état de chlorhydrate, avec:
produit AAB....................0,03 g
acide chlorhydrique 0,1N..........0,3 cm3
eau distillée.................qsp 3 $cm^3$

Le diméthylamino-3 méthyl-2 propanethiol peut être préparé de la manière suivant:

A une solution de 5,33 g de N,N-diméthyl acétylthio-3 méthyl-2 propylamine dans 50 $cm^3$ de méthanol anhydre, on ajoute 0,026 g de sodium. Le mélange obtenu est chauffé au reflux pendant 7 heures puis le méthanol est éliminé sous pression réduite (2,7 kPa) à 50° C. Le résidu est distillé sous pression réduite (2,7 kPa) On obtient ainsi 0,9 g de diméthylamino-3 méthyl-2 propanethiol sous forme d'une huile jaune distillant à 56° C sous 2,7 kPa.

La N,N-diméthyl acétylthio-3 méthyl-2 propylamine peut être préparée de la manière suivante:

A une solution de 29,5 g de N,N-diméthyl chloro-1 méthyl-2 propylamine dans 120 $cm^3$ d'isopropanol, on ajoute 15,7 $cm^3$ d'acide thiolacétique. Le mélange obtenu est chauffé au reflux pendant 48 heures, puis l'isopropanol est éliminé sous pression réduite (2.7 kPa) à 60° C. Le résidu obtenu est traité par 100 $cm^3$ d'une solution aqueuse saturée de bicarbonate de sodium et la phase aqueuse est extraite 3 fois avec 600 $cm^3$ au total d'éther éthylique. Les phases organiques sont réunies, séchées sur du sulfate de magnésium, filtrées et concentrées à sec. Le résidu obtenu est purifié par chromatographie "flash" [éluant: chloroforme-méthanol (90-10 en volumes)] , les fractions 6 à 10 sont réunies et concentrées à sec sous pression réduite (2,7 kPa) à 30° C. On obtient ainsi 5,57 g de N,N-diméthyl acétylthio-3 méthyl-2 propylamine sous forme d'une huile rouge.

La N,N-diméthyl chloro-1 méthyl-2 propylamine peut être préparée selon la méthode décrite par J.P. BOURQUIN et coll., Helv. Chim. Acta, 41, 1072 (1958).

**Exemple d'application 23**

En opérant d'une manière analogue à celle décrite à l'exemple d'application 18, mais à partir de 4 g de diméthylamino-méthylène-5δ pristinamycine $I_A$ et 1,14 g de diméthylamino-2 méthyl-2 propanethiol et après purification par chromatographie "flash" [éluant: chloroforme-méthanol (92-8 en volumes)] et concentration à sec des fractions 12 à 30 sous pression réduite (2,7 kPa) à 30° C, on obtient 1,4 g de (diméthylamino-2 méthyl-2 propyl)thiométhylène-5δ pristinamycine $I_A$ aous forme d'une poudre beige fondant vers 200° C.

Spectre RMN:
0,55 (dd, 1H x 0,20: 5$\beta_2$ 2ème isomère)
0,68 (dd, 1H x 0,80: 5$\beta_2$ ler isomère)
1,15 (s, 6H: -C(C$\underline{H}_3$)$_2$-)
2,30 (s, 6H x 0,80: -N($CH_3$)$_2$ ler isomère)
2,42 (s, 6H x 0,20: -N($CH_3$)$_2$ 2ème isomère)
2,40 (d, 1H: 5$\beta_1$)
2,80 (sous un massif: -S-C$\underline{H}_2$-)
3,42 (dd, 1H: 5$\varepsilon_2$)
7,55 (s large, 1H: -C$\underline{H}$-S)
7,80 (dd, 1H x 0,80: 1'$H_6$ ler isomère)
7,98 (dd, 1H x 0,20: 1'$H_6$ 2ème isomère)

On obtient une solution aqueuse à 1 % de (diméthylamino-2 méthyl-2 propyl) thiométhylène-5δ pristinamycine $I_A$ (produit AAC), à l'état de chlorhydrate, avec
produit AAC .......................... 0,03 mg
acide chlorhydrique ............... 0,3 ml
eau distillée ................... qsp 3 ml

Le diméthylamino-2 méthyl-2 propanethiol peut être réparé selon la méthode décrite par H.R. SNYDER, J.M. STEWART, J.B. ZIEGLER, J. Am. Chem. Soc., 69, 2672 (1947).

**Exemple d'application 24**

En opérant d'une manière analogue à celle décrite à l'exemple d'application 18, mais à partir de 4 g de diméthylamino-méthylène-5δ pristinamycine $I_A$ et 1,1 g de (pyrrolidinyl-l)-2 éthanethiol et après purification par chromatographie "flash" [éluant: chloroforme-méthanol (96-4 en volumes)] et concentration à sec des fractions

9 à 15 sous pression réduite (2,7 kPa) à 30°C, on obtient 1,3 g de [(pyrrolidinyl-l)-2 éthyl] thiométhylène-5δ pristinamycine $I_A$ aous forme d'une poudre beige fondant vers 180°C.

Spectre RMN:

0,65 (dd, 1H: $5\beta_2$)

1,85 (m, 4H:

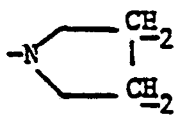

)

2,45 (d, 1H: $5\beta_1$)

2,75 et 2,90 (m, 8H: -C$\underline{H}_2$-N

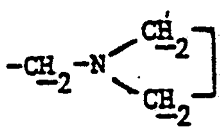

et -$SCH_2$-)

3,43 (dd' 2H: 5ε)

7,60 (s large, 1H: =C$\underline{H}$-S-)

7,85 (dd, 1H: 1'$H_6$)

On obtient une solution aqueuse à 1 % de á(pyrrolidinyl-l)-2 éthyl] thiomethylène-5δ pristinamycine $I_A$ (produit AAD), à l'état de chlorhydrate, avec:

produit AAD .......................... 0,03 g

acide chlorhydrique 0,1N ............ 0,3 ml

eau distillée ...................qsp 3 ml

Le (pyrrolidinyl-l)-2 éthanethiol peut être préparé selon la méthode décrite par J.W. HAEFFELE, R.W. BROGE, Proc. Sci. Toilet Goods Assoc. 32, 52 (1959) [Chem. Abstr. 54, 17234e (1960)].

**Exemple d'application 25**

En opérant d'une manière analogue à celle décrite à l'exemple d'application 18, mais à partir de 4 g de diméthylaminométhylène-5δ pristinamycine $I_A$ et 1,74 g de (méthyl-1 pyrrolidinyl-2)-2 éthanethiol et après purification par chromatographie "flash" [éluant: chloroforme-méthanol (92-8 en volumes)] et concentration à sec des fractions 12 à 22 sous pression réduite (2,7 kPa) à 30°C, on obtient 1,33 g de [(méthyl-1 pyrrolidinyl-2)-2 éthyl] thiométhylène-5δ pristinamycine $I_A$ sous forme d une poudre beige fondant vers 215°C.

Spectre RMN:

0,65 (dd, 1H: $5\beta_2$)

1,4-2,3 (m, 6H:

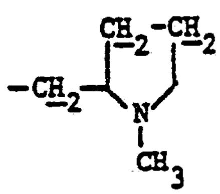

)

2,40 (d, 1H: $5\beta_1$)

2,48 (s, 3H:

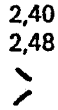

N-C$\underline{H}_3$ pyrrolidine)

3,40 (dd, 1H: $5\varepsilon_2$)

7,50 (s, large, 1H: =C$\underline{H}$-)

7,80 (dd, 1H x 0,85: 1'$H_6$ ler isomère)

8,00 (dd, 1H: x 0,15: 1'$H_6$ 2ème isomère)

On obtient une solution aqueuse à 0,6 % de [(méthyl-1 pyrrolidinyl-2)-2 éthyl] thiométhylène-5δ pristinamycine $I_A$ (produit AAE), à l'état de chlorhydrate, avec:

produit AAE......................0,03 g

acide chlorhydrique 0,1N ............... 0,3 $cm^3$

eau distillée ................qsp 5 $cm^3$

Le méthyl-1 (pyrrolidinyl-2)-2 éthanethiol peut être préparé d'une manière analogue à celle décrite à l'exemple d'application 22 pour préparer le diméthylamino-3 méthyl-2 propanethiol, mais à partir de 15,7 g d'(acétylthio-2 éthyl)-2 méthyl-1 pyrrolidine et 0,07 g de sodium. On obtient ainsi 12,2 g de produit sous forme d'une huile rouge.

L'(acétylthio-2 éthyl)-2 méthyl-1 pyrrolidine peut être préparée d'une manière analogue à celle décrite à l'exemple d'application 22 pour préparer la N,N-diméthylacéthylthio-3 méthyl-2 propylamine mais à partir de 12,7 g de (chloro-2 éthyl)-2 méthyl-1 pyrrolidine et 6,8 $cm^3$ d'acide thiolacétique. On obtient ainsi 15,7 g de produit sous forme d une huile rouge.

**Exemple d'application 26**

En opérant d'une manière analogue à celle décrite à l'exemple d'application 18, mais à partir de 3,0 g de diméthylamino-méthylène-5δ pristinamycine $I_A$ et 0,48 g de mercapto-4 méthyl-1 pipéridine et après purification par chromatographie "flash" [éluant: chloroforme-méthanol (90-10 en volumes)] et concentration à sec des fractions 15 à 19 sous pression réduite (2,7 kPa) à 30°C, on obtient 1,2 g de (méthyl-1 pipéridyl-4) thiométhylène-5δ pristinamycine $I_A$ sous forme d'une poudre jaune fondant vers 170°C.

Spectre RMN:
0,68 (dd, 1H: $5\beta_2$)
2,0-2,2 (m, 4H:
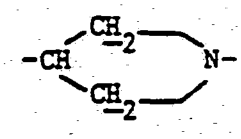 )
2,30 (s, 3H:
$>N-CH_3$)
2,45 (d, 1H: $5\beta_1$)
2,85 (m, 4H:
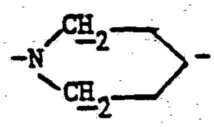 )
3,05 (mt, 1H:
$-S-CH-$ )
3,40 (dd, 1H: $5\varepsilon_2$)
5,15 (d, 1H: $5\varepsilon_1$)
7,67 (s large, 1H: =CH-S-)
7,85 (dd, 1H x 0,85: 1'$H_6$ ler isomère)
8,0 (dd, 1H x 0,15: 1'$H_6$ 2ème isomère)

On obtient une solution aqueuse à 1 % de (méthyl-1 pipéridyl-4) thiométhylène-5δ pristinamycine $I_A$ (produit AAF), à l'état de chlorhydrate, avec:
produit AAF ...................... 0,05 g
acide chlorhydrique 0,1N ......... 0,5 $cm^3$
eau distillée ................qsp 5 $cm^3$

La mercapto-4 méthyl-1 pipéridine peut être préparée selon la méthode décrite par H. BARRER et R.E. LYLE, J. Org. Chem. 27, 641 (1962)

**Exemple d'application 27**

En opérant d'une manière analogue à celle décrite à l'exemple d'application 18, mais à partir de 4 g de diméthylaminométhylène-5δ pristinamycine $I_A$ et 0,8 g d'éthyl-1 mercapto-3 pipéridine et après purification par chromatographie "flash" [éluant: chloroformeméthanol (92-8 en volumes)] et concentration à sec des fractions 6 à 9 sous pression réduite (2,7 kpa) à 30°C, on obtient 1,1 g d'(éthyl-1 pipéridyl-3) thiométhylène-5δ pristinamycine $I_A$ fondant vers 175°C.

Spectre RMN:
0,70 (s large, 1H: $5\beta_2$)
1,20 (t, 3H: $-CH_2C\underline{H_3}$)
2,45 (d large, 1H: $5\beta_1$)
2,90 (m, 6H:

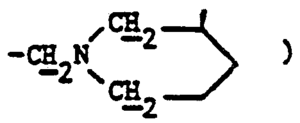

7,50 (s large, 1H: =CH-S-)
7,80 (dd, 1H x 0,80: 1'$H_6$ Ier isomère)
7,95 (dd, 1H x 0,20: 1'H6 2ème isomère)

On obtient une solution aqueuse à 1 % d'(éthyl-1 pipéridyl-3) thiométhylène-5δ pristinamycine $I_A$ (produit AAC), à l'état de chlorhydrate, avec:
produit AAG ........................ 0,03 g
acide chlorhydrique 0,1N ........... 0,3 $cm^3$
eau distillée .................. qsp 3 $cm^3$

L'éthyl-1 mercapto-3 pipéridine peut être préparée selon la méthode décrite par J.H. BIEL et coll., J. Am. Chem.Soc. 77, 2250 (1955).

**Exemple d'application 28**

En opérant d'une manière analogue à celle décrite à l'exemple d'application 18, mais à partir de 3,0 g de diméthylamino-méthylène-5δ pristinamycine $I_A$ et 0,55 g de N-(mercapto-2 éthyl) N,N',N'-triméthyléthylènediamine et après purification par chromatographie "flash" [éluant: chloroforme-méthanol (90-10 en volumes)] et concentration à sec des fractions 17 à 24 sous pression réduite (2,7 kPa) à 30° C, on obtient 1,0 g de [(diméthylamino-2 éthyl) méthylamino-2 éthyl] thiométhylène-5δ pristinamycine $I_A$ sous forme d'une poudre jaune fondant vers 160° C.
Spectre RMN:
0,68 (dd, 1H: $5\beta_2$)
2,30 (s, 3H:

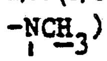

2,40 (d, 1H: $5\beta_1$)
2,4-3,1 (m, 8H: $-S(C\underline{H_2})_2N-(C\underline{H_2})_2$

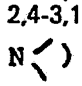

3,40 (dd, 1H: $5\varepsilon_2$)
5,10 (sous un massif, 1H: $5\varepsilon_1$)
7,58 (s large, 1H: "C$\underline{H}$-S-)
7,80 (dd, 1H: 1'$H_6$)

On obtient une solution aqueuse à 1 % de [(diméthyl-amino-2 éthyl) méthylamino-2 éthy] thiométhyléne-5δ pristinamycine $I_A$ (produit AAH), avec:
produit AAH ............................ 0,03 g
eau distillée .....................qsp 3 $cm^3$

La N-(mercapto-2 éthyl) N,N',N -triméthyléthylèdnediamine peut être préparée de la manière suivante: on ajoute 5,0 g de carbonate d'éthyle et de mercapto-2 éthyle à une solution de 10,2 g de N,N',N'-triméthyléthylènediamine dans 40 $cm^3$ de toluène portée au reflux. Après 5 heures à reflux, le toluène est éliminé sous pression réduite (2,7 kPa) à 50° C et le résidu distillé à cette pression. On obtient la N-(mercapto-2 éthyl) N,N',N'-triméthyléthylènediamine sous forme d'un liquide jaune distillant à 105° C sous 2,7 kPa.

Le carbonate d'éthyle et de mercapto-2 éthyle peut être préparé selon la méthode décrite par D.D REYNOLDS, D.L. FIELDS, D.L. JOHNSON, J. Org. Chem., 26, 5125 (1961).

**Exemple d'application 29**

En opérant d'une manière analogue à celle décrite à l'exemple d'application 18, mais à partir de 4 g de diméthylaminométhylè-ne-5δ pristinamycine $I_A$et 2 g de bis(diméthylamino)-1,3 propane thiol-2 et après purification par chromatographie "flash" [éluant: chloroforme-méthanol (92-8 en volumes)] et concentration à sec des fractions 32 à 56 sous pression réduite (2,7 kPa) à 30° C, on obtient 1,6 g de [bis(diméthylamino)-1,3

propyl-2] thiométhylène-5δ pristinamycine $I_A$ sous forme d'une poudre beige fondant vers 190°C.

Spectre RMN:

0,55 (dd, 1H x 0,80: $5\beta_2$ 2ème isomère)

0,67 (dd, 1H x 0,20: $5\beta_2$ ler isomère)

2,30 (m, 6H: $-N(C\underline{H}_3)_2$)

2,8-3,2 (m, 4H: $-S-CH(C\underline{H}_2$

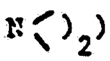

7,62 (m, 1H: $-C\underline{H}-S-$)

7,80 (dd, 1H x 0,80: $1'H_6$ ler isomère)

7,98 (dd, 1H x 0,20: $1'H_6$ 2ème isomère)

On obtient une solution aqueuse à 1 % de [bis(diméthyl-amino)-1,3 propyl-2] thiométhylène-5δ pristinamycine $I_A$ (produit AAI), à l'état de chlorhydrate, avec:

produit AAI .......................... 0,03 g

acide chlorhydrique 0,1N ............ 0,3 $cm^3$

eau distillée .................. qsp 3 $cm^3$

Le bis(diméthylamino)-1,3 propanethiol-2 peut être préparé selon la méthode décrite par J.M. Stewart, J. Org. Chem., 29, 1655 (1964).

**Exemple d'application 30**

En opérant d'une manière analogue à celle décrite à l'exemple d'application 18, mais à partir de 3,0 g de diméthylamino-méthylène-5δ pristinamycine $I_A$ et 0,58 g de (mercapto-2 éthyl)-1 méthyl-4 pipérazine et après purification par chromatographie "flash" [éluant chloroforme-méthanol (87,5-12,5 en volumes)] et concentration à sec des fractions 16 à 30 sous pression réduite (2,7 kPa) à 30°C, on obtient 1,6 g de [(méthyl-4 pipérazinyl-1)-2 éthyl] thiométhylène-5δ pristinamycine $I_A$ sous forme d'une poudre beige fondant vers 170°C.

Spectre RMN:

0,56 (dd, 1H x 0,20: $5\beta_2$ ler isomère)

0,68 (dd, 1H x 0,80: $5\beta_2$ 2ème isomère)

2,40 (s, 3H:

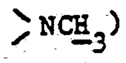

2,5-3 (m, 12H: $-S(C\underline{H}_2)_2$

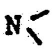

+ tous les $-C\underline{H}_2-$de pipérazine)

3,42 (dd, 1H: $5\varepsilon_2$)

5,12 (d large: $5\varepsilon_1$)

7,60 (s large, 1H: $=C\underline{H}S-$)

7,80 (dd, 1H: $1'H_6$, mélange des 2 isomères)

On obtient une solution aqueuse à 1 % de [(méthyl-4 pipérazinyl-1)-2 éthyl] thiométhylène-5δ pristinamycine $I_A$ (produit AAH), à l'état de chlorhydrate, avec:

produit AAJ ........................ 0,05

acide chlorhydrique 0,1N .............. 0,5 $cm^3$

eau distillée ............. ...... qsp 5 $cm^3$

La(mercapto-2 éthyl)-1 méthyl-4 pipérazine peut être préparée selon la méthode décrite par D.D. REYNOLDS et coll., J. Org. Chem. 26, 5125 (1961).

**Exemple d'application 31**

En opérant d'une manière analogue à celle décrite à l'exemple d'application 18, mais à partir de 4,0 g de diméthylaminométhy-lène-5δ pristinamycine $I_A$ et 1,5 g de (mercapto-3 propyl)-1 méthyl-4 pipérazine et après purification par chromatographie "flash" [éluant: chloroforme-méthanol (95-5 en volumes)] et concentration à sec des fractions 24 à 41 sous pression réduite (2,7 kPa) à 30°C, on obtient 2,06 g de [(méthyl-4 pipérazinyl-1)-3 propyl] thiométhylène-5δ pristinamycine $I_A$ sous forme d'une poudre beige fondant vers 190°C.

Spectre RMN:

0,68 (dd, 1H: $5\beta_2$)

1,90 (mt 2H: $-CH_2-C\underline{H}_2CH_2$

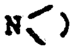

2,40 (s, 3H:

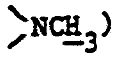

2,3 à 2,8 (m, 8H: -S-C$\underline{H}_2$- + -C$\underline{H}_2$-

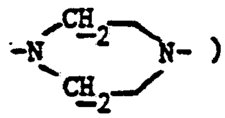

3,45 (m 1H: 5$\varepsilon_2$)
7,64 (s large, 1H x 0,80: =CH-S- ler isomère)
7,70 (s large, 1H x 0,20: =CH-S- 2ème isomère)
7,80 (dd, 1H x 0,80: 1'$H_6$ ler isomère)
7,98 (dd, 1H x 0,20: 1'$H_6$ 2ème isomère)

On obtient une solution aqueuse à 10 % de [(méthyl-4 pipérazinyl-1)-3 propyl] thiométhylène-5δ pristinamycine $I_A$ (produit AAK), à l'état de chlorhydrate, avec:

produit AAK .......................... 0,05 g
acide chlorhydrique 0,1N .......... qsp 0,5 ml

La (mercapto-3 propyl)-1 méthyl-4 pipérazine peut être préparée d'une manière analogue à celle décrite dans l'exemple d'application 22 pour préparer le diméthylamino-3 méthyl-2 propanethiol, mais à partir de 109 g d"(acétylthio-3 propyl)-1 méthyl-4 pipérazine et 0,46 g de sodium. On obient 64,8 g de (mercapto-3 propyl)-1 méthyl-4 pipérazine sous forme d'une huile jaune distillant à 133° C sous 0,13 kPa

L'(acétylthio-3 propyl)-1 méthyl-4 pipérazine peut être préparée d'une manière analogue à celle décrite dans l'exemple d'application 22 pour préparer la N,N-diméthylacétylthio-3 méthyl-2 propylamine, mais à partir de 138 g de (chloro-3 propyl)-1 méthyl-4 pipérazine et 68,5 g d'acide thiolacétique. Or obtient ainsi 109 g d'(acétylthio-3 propyl)-1 méthyl-4 pipérazine sous forme d'une huile jaune distillant vers 160° C sous 0,13 kPa.

**Exemple d'application 32**

En opérant d'une manière analogue à celle décrite à l'exemple d application 18, mais à partir de 4,0 g de diméthylamino-méthyléne-5δ pristinamycine $I_A$ et 1,3 g d'iodure de mercapto-3 méthyl-2 propylammoniun et après purification par chromatographie "flash" (éluant: chloroforme-méthanol (80-20 en volumes)] et concentration à sec des fractions 12 à 22 sous pression réduite (2,7 kPa) à 30°C, on obtient 1,05 g d'iodure de (méthyl-2 triméthylammonio-3 ppropyl) thiomethyléne -5δ pristinamycine $I_A$ sous forme d'une poudre ocre fondant vers 150° C.

Spectre de RMN:
1,05 - 1,35 (m, 8H: lγ + 3$\gamma_2$ + 3β +
-C$\underline{H}$-C$\underline{H}_3$)
2,40 (m, 2H 5$\beta_1$ +
-C$\underline{H}$-CH$_3$)
2,90 (mt, 3H: 4$\beta_2$ + -S-C$\underline{H}_2$-)
3,20 (mt, 7H: 4NCH$_3$ + 4$\beta_1$ + 3$\delta_1$ + -C$\underline{H}_2$N$^{\oplus}$(CH$_3$)$_3$)
3,40 (mt, 9H: -N$^{\oplus}$(C$\underline{H}_3$)$_3$)

On obtient une solution aqueuse à 1 % de (méthyl-2 triméthylammonio-3 propyl) thiométhylène -5δ pristinamycine $I_A$ iodure, (produit AAL), à l'état de chlorhydrate, avec:

produit AAL ...................0,02 g
acide chlorhydrique 0,1N ............ 0,2 cm$^3$
eau distillée ..................qsp 2 cm$^3$

L'iodure de mercapto-3 méthyl-2 propylammonium peut être préparé de la manière suivante: on ajoute 0,024 g de méthylate de sodium à une solution de 3,6 g d'iodure d'acétylthio-3 méthyl-2 propylammonium dans 18 cm 3 de méthanol à une température voisine de 20°C. Le mélange obtenu est porté au reflux pendant 1 heure puis laissé à température ambiante pendant 16 heures. Le méthanol est éliminé sous pression réduite (2,7 kPa) à 50°C Le résidu est agité pendant 1 heure avec 35 cm$^3$ d'isopropanol et la suspension blanche est filtrée puis séchée. On obtient ainsi 3,1 g d'iodure de mercapto-3 méthyl-2 propylammonium sous forme d'une poudre beige fondant à 120°C.

L'iodure d'acétylthio-3 méthyl-2 propylanmonium peut être préparé de la manière suivante: on ajoute 1,4 cm$^3$ d'iodure de méthyle à une solution de 3,5 g de N,N-diméthyl acétylthio-3 méthyl-2 propylamine dans 35 cm$^3$

d'acétonitrile; après 18 heures d'agitation à une température voisine de 20°C, le précipité est filtré puis séché. On obtient ainsi 3,8 g d'iodure d'acétylthio-3 méthyl-2 propylammonium sous forne d'une poudre blanche fondant à 181°C.

**Exemple d'application 33**

En opérant d'une manière analogue à celle décrite à l'exemple d'application 18, mais à partir de 1,84 g de diméthylaminométhylè-ne-5δ pristinamycine $I_A$ et 3,28 g de sel de sodium de l'acide mercapto-2 éthanesulfonique et après purification par chromatographie "flash" [éluant: chlorure de méthylène-méthanol (90-10 en volumes)] et concentration à sec des fractions 6 à 14 sous pression réduite (2,7 kPa) à 30°C, on obtient 0,8 g d'(hydroxysulfonyl-2 éthyl) thiométhylène-5δ pristinamycine $I_A$ sous forme d'une poudre jaune fondant à une température supérieure à 280°C.

Le spectre infra-rouge comporte les bandes caractéristiques des pristinamycines: 1745, 1680, 1650, 1525, 815, 740 et 705 $cm^{-1}$, plus les bandes caractéristiques du groupe $-SO_3H$ [1200 cm (large) et 1050 $cm^{-1}$].

On obtient une solution aqueuse à 5 % d'(hydroxysulfonyl-2 éthyl) thiométhylène-5δ pristinamycine $I_A$ (produit AAM), avec:

produit AAM .......................... 0,1 g
eau distillée ....................qsp 2 $cm^3$

**Exemple d'application 34**

A une solution de 0,87 g de (mercapto-2 propyl)-1 méthyl-4 pipérazine dans 50 $cm^3$ d'éthanol additionnée de 0,34 g d'éthylate de sodium, on ajoute une solution de 5,2 g de (méthyl-4 phényl) sulfonyloxyméthylène-5δ pristinamycine $I_A$ dans 50 $cm^3$ de chlorure de méthylène. Le mélange réactionnel est agité pendant 16 heures à une température voisine de 20°C puis dilué avec 500 $cm^3$ de chlorure de méthylàne et 100 $cm^3$ d'eau distillée. Après agitation, la phase aqueuse est extraite 2 fois par 50 $cm^3$ de chlorure de méthylène au total. Les phases organiques sont réunies, séchées sur du sulfate de magnésium, filtrées puis concentrées à sec sous pression réduite (2,7 kPa) à 30°C. Le résidu est purifié par chromatographie "flash" [éluant: chloroforme-méthanol (97,5-2,5 en volumes)]. Les fractions 33 à 80 sont réunies et concentrées à sec sous pression réduite (2,7 kPa) à 30°C. On obtient ainsi 1,25 g de [(méthyl-4 pipérazinyl-1)-3 propyl-2] thiométhylène-5δ pristinamycine $I_A$ sous forme d'une poudre beige fondant vers 195°C.

Spectre RMN:
0,70 (dd, 1H: $5\beta_2$)
1,25 (d, 3H:
$-\underset{|}{C}H-\underline{CH_3}$)
2,30 (s, 3H:
$>N-\underline{CH_3}$)
2,50 (m, 10H: $-C\underline{H_2}$
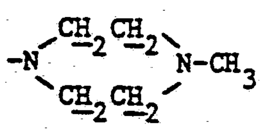
)
3,40 (dd, 1H: $5\varepsilon_2$)
7,85 (dd large, 1H: $1'H_6$)

On obtient une solution aqueuse à 10 % de [(méthyl-4 pipérazinyl-1)-3 propyl-2] thiométhyléne-5δ pristinamcine $I_A$ (produit AAN) sous forme de chlorhydrate, avec:

produit AAN .......................... 0,03 g
acide chlorhydrique 0,1N ........... 0,3 $cm^3$

La (mercapto-2 propyl)-1 méthyl-4 pipérazine est préparée en chauffant à 100°C pendant 16 heures un mélange de 19 $cm^3$ de sulfure de propylène et de 29 $cm^3$ de N-méthylpipérazine On obtient ainsi 32 g d'une huile incolore distillant à 105°C sous 1,3 kPa.

**Exemple d'application 35**

En opérant d'une manière analogue à celle décrite à l'exemple d'application 34, mais à partir de 5,2 g de (méthyl-4 phényl)sulfo-nyloxyméthylène-5δ pristinamycine $I_A$, 0,6 g de diméthylamino-1 propa-nethiol-2 et 0,34 g d'éthylate de sodium et après purification par chromatographie "flash" [éluant: chloroforme-méthanol) 95-5 en volumes)] et concentration à sec des fractions 16 à 38 sous pression réduite (2,7 kPa) à 30° C, on obtient 1 g de (diméthylamino-3 propyl-2) thiométhylène-5δ pristinamycine $1_A$ sous forme d'une poudre jaune fondant à 172° C.

Spectre RMN:
0,65 (dd, 1H: 5$\beta_2$)
1,10 (d, 3H:
$-\overset{|}{C}H-C\underline{H}_3$)
2,30 (s, 6H: $-N(C\underline{H}_3)_2$)
7,60 (s large, 1H: $=C\underline{H}-S-$)
7,85 (dd, 1H: 1'$H_6$)

On obtient.une solution aqueuse à 5 % de (diméthylamino-3 propyl-2) thiométhylène-5δ pristinamycine $I_A$ (produit AAO), à l'état de chlorhydrate, avec:
produit AAO ........................ 0,03 g
acide chlorhydrique 0,1N ........... 0,3 $cm^3$
eau distillée .................qsp 0,6 $cm^3$

Le diméthylamino-1 propanethiol-2 peut être préparé selon la méthode décrite par S.D. TURK et coll., J. Org. Chem. 29, 974 (1964)

**Exemple d'application 36**

En opérant d'une manière analogue à celle décrite à l'exemple d'application 34, mais à partir de 6,3 g de (méthyl-4 phényl)sul-fonyloxyméthylène-5δ pristinamycine $I_A$, de 1,05 g de diéthylamino-5 pentanethiol-2 et o,408 g d'éthylate de sodium et après purification par chromatographie "flash" [éluant: chloroforme-méthanol (97,5-2,5 en volumes)[ et concentration à sec des fractions 47 à 65 sous pression réduite (2,7 kPa) à 30 C, on obtient 1,32 g de (diéthylamino-5 pentyl-2) thiométhylène-5δ pristiniamycine $I_A$ sous forme d'une poudre beige fondant vers 185° C.

Spectre RMN:
0,65 (d, 1H: 5$\beta_2$)
1,20 (t, 6H: $-(CH_2C\underline{H}_3)_2$)
1,40 (d, 3H:
$-\overset{|}{C}H-C\underline{H}_3$)
1,70 (s, large, 4H: $-CH(C\underline{H}_2)_2-CH_2$
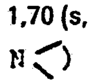
2,65 (q, 4H: $-N(C\underline{H}_{2>l-CH_3})_2$)
3,50 (dd, 1H: 5$\varepsilon_2$)
7,65 (s large, 1H: $=C\underline{H}-S-$)
7,85 (dd, 1H: 1'$H_6$

On obtient une solution aqueuse à 10 % de (diéthylamino-5 pentyl-2) thiométhylène-5δ pristinamycine $I_A$ (produit AAP), sous form de chlorhydrate, avec:
produit AAP ............................. 0,05 g
acide chlorhydrique 0,1N .................... 0,5 $cm^3$

Le diéthylamino-5 pentanethiol-2 peut être préparé d'une manière analogue à ceile décrite à l'exemple d'application 22 pour préparer le diméthylamino-3 méthyl-2 propanethiol, mais à partir de 4,0 g de N,N-diéthylacétylthio-4 pentanamine-1 et 0,046 g de sodium. Après purification par chromatographie "flash" [éluant: acétate d'éthyle-méthanol (70-30 en volumes) et concentration à sec des fractions 16 à 24, on obtient 2,0 g de diéthylamino-5 pentanethiol-2 sous forme d'une huile jaune.

La N,N-diethylacétylthio-4 pentanamine-1 peut être préparée d'une maniére analogue à celle decrite à l'exemple d'application 22 pour préparer la N,N-diméthylacétylthio-5 méthyl-2 propylamine, mais à partir de 32 g de N,N-diéthylchloro-4 pentanamine-1 et 15,2 g d'acide thiolacérique. On obtient ainsi 4,31 g de produit sous forme d'une huile jaune.

La N,N-diéthylchloro-4 pentanamine-1 peut être préparée selon la méthode décrite par M.S. KHARASH et C.F. FUCHS, brevet US 2 432 905.

**Exemple d'application 37**

Une solution de 7,6 g de [(méthyl-4 phényl) sulfonyloxy-méthylène]-5δ pristinamycine $I_A$ dans 60 $cm^3$ de tétrahydrofuranne est refroidie à une température voisine de -10°C. On y ajoute lentement en maintenant cette température une solution de 0,65 g de diméthylamino-2 éthanol dans 60 $cm^3$ e tétrahydrofuranne additionnée de 0,35 g d'une dispersion à 50 % d'hydrure de sodium dans l'huile minérale. A la fin de l'addition on laisse remonter lentement la température au voisinage de 20°C. Le mélange réactionnel est agité pendant 24 heures à cette température puis dilué avec 500 $cm^3$ de chlorure de méthylène et lavé 2 fois avec 50 $cm^3$ d'une solution saturée de chlorure d'ammonium. La phase organique est séchée sur du sulfate de magnésium, filtrée puis concentrée à sec sous pression réduite (2,7 kPa) à 40°C. Le résidu obtenu est purifié par chromatographie "flash" [éluant: chloroforme-métahnol (95-5 en volumes)]. Les fractions 12 à 17 sont réunies et concentrées à sec sous pression réduite (2,7 kPa) à 25°C. On obtient ainsi 1,5 g de (diméthylamino-2 éthoxyméthylène)-5δ pristinamycine $I_A$ sous forme d'une poudre beige fondant vers 160°C.

Spectre RMN:

0,65 (dd, 1H: $5\beta_2$)
2,3 (s, 6H: -N($\underline{H_3}$)$_2$)
2,65 (m, 2H: -c$\underline{H_2}$
N<)
3,42 (dd, 1H: $5\varepsilon_2$)
4,15 (t, 2H: -OC$\underline{H_2}$-)
5,15 (d, 1H: $5\varepsilon_1$)
7,45 (sous les aromatiques, 1H:

>C=C$\underline{H}$O-)

7,80 (dd, 1H: 1'$H_6$)

On obtient une solution aqueuse à 1 % de (diméthylamino-2 éthoxyméthylène)-5δ pristinamycine $I_A$ (produit AAQ), sous forme de chlorhydrate, avec:

produit AAQ ...................... 0,03 g
acide chlorhydrique 0,1N .......... 0,3 $cm^3$
eau distillée ................ qsp 3 $cm^3$

**Exemple d'application 38**

A une solution de 0,5 g de (méthyl-4 phényl)sulfonyloxy méthylène-5δ pristinamycime $I_A$ dans 25 $cm^3$ d'éthanol, on ajoute 0,12 g d'amino-4 méthyl-1 pipéridine à une température voisine de 20°C. Après 16 heures d'agitation à cette température, le mélange réactionnel est dilué avec 100 $cm^3$ de chlorure de méthylène, lavé deux fois avec 100 $cm^3$ au total d'eau distillée. La phase organique est séchée sur sulfate de sodium puis concentrée sous pression réduite (2,7 kPa) à 30°C. Le résidu est agité avec 15 $cm^3$ d'éther éthylique. Après filtration, on obtient 0,42 g de (méthyl-1 pipéri-dyl-4) aminométhylène-5δ pristinamycine $I_A$ sous forme d'une poudre blanche dont les caractéristiques sont identiques à celles décrites à l'exemple d'application 14.

L'amino-4 méthyl-1 pipéridine peut être préparée comme indiqué à l'exemple d'application 14.

**Exemple d'application 39**

A partir des produits de formule générale (I) décrits dans les exemples 3, 5, 6, 8, 10, 11, 12, 13, 14, 15, 21 22 et 24 ont été préparées la (méthyl-1 pipéridyl-4) aminométhylène-5δ pristinamycine $I_A$ décrite à l'exemple d'application 14 (ci-après désignée par "Amine d'application 14") et la (diméthylamino-3 propyl) thiométhyléne-5δ pristinamycine $I_A$ décrite à l'exemple d'application 20 (ci-aprés désignée par "Thiol d'application 20"). Les conditions opératoires sont indiquées dans le tableau suivant:

| Produit de départ (référence de l'exemple) | Conditions de la réaction (Solvant, Température, Durée) | Produit préparé |
|---|---|---|
| 3 | $CH_3COOH$, 20°C, 20 heures | Amine d'application 14 |
| 5 | $CH_3COOH/CF_3COOH$ 20°C, 20 heures | Thiol d'application 20 |
| 6 | $CH_3COOH$, 20°C, 20 heures | Amine d'application 14 |
| 8 | $CH_3COOH/CF_3COOH$, 20°C, 48 heures | Amine d'application 14 |
| 11 | $CH_3COOH/CF_3COOH$, 20°C, 20 heures | Thiol d'application 20 |
| 12 | $CH_3COOH/CF_3COOH$, 20°C, 48 heures | Thiol d'application 20 |
| 13 | $CH_3COOH$, 20°C, 20 heures | Thiol d'application 20 |
| 14 | $CH_3COOH$, 20°C, 10 jours | Amine d'application 14 |
| 15 | $CH_3COOH/CF_3COOH$, 20°C 20 heures | Thiol d'application 20 |
| 21 | $CH_3COOH$, 20°C, 6 heures | Amine d'application 14 |
| 22 | $CH_3COOH$, 20°C, 20 heures | Amine d'application 14 |
| 24 | $C_2H_5OH$, 20°C, 20 heures | Thiol d'application 20 |

En thérapeutique humaine, les produits de formule générale (IX) sont particulièrement utiles dans le traitement des infections d'origine bactérienne.

Les doses dépendent de l'effet recherché et de la durée du traitement; pour un adulte, elles sont généralement comprise entre 2000 et 4000 mg par jour par voie parentérale, particulièrement par voie intraveineuse en perfusion lente.

D'une façon générale, le médecin déterminera la posologie qu'il estime la plus appropriée en fonction de l'age, du poids et de tous les autres facteurs propres au sujet à traiter.

**Revendications**

pour les Etats contractants BE CH DE FR GB IT LI LU NL SE

1 - Nouveau dérivé de synergistine, caractérisé en ce qu'il répond à la formule générale:

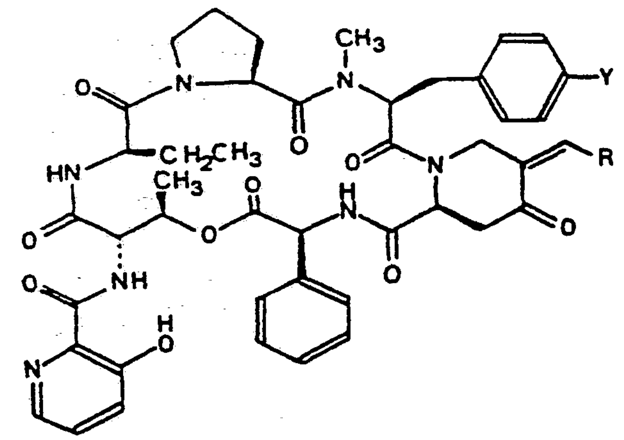

dans laquelle Y représente un atome d'hydrogène ou un radical diméthylamino et R représente:
a) soit un atome d'hydrogène ou un radical hydroxy,
b) soit un radical de formule générale:

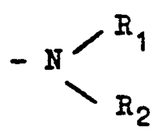

dans laquelle $R_1$ et $R_2$, idemtiques ou différents, représentent un atome d'hydrogène ou un radical phényle ou pyridyle (éventuellement substitués par un radical dialcoylamino dont la partie alcoyle contient 1 à 4 atomes de carbone en chaîne droite ou ramifiée) ou un radical alcoyle contenant 1 à 10 atomes de carbone en chaîne droite ou ramifiée éventuellement substitué [par un radical hydroxy, mercapto, carboxy, pyridyle, anilino, alcoylamino ou dialcoylamino dont au moins l'une des parties alcoyle est elle-même substituée par un radical hydroxy, mercapto, carboxy ou anilino], ou radical alcényle contenant 3 ou 4 atomes de carbone, ou un radical alcynyle contenant 3 ou 4 atomes de carbone, ou bien $R_1$ et $R_2$ forment ensemble avec l'atome d'azote auquel ils sont liés un hétérocycle à 5 ou 6 chaînons contenant éventuellement un autre hétéroatome choisi parmi l'oxygène, le soufre ou l'azote (éventuellement substitué par un radical alcoyle),

c) soit un atome d'halogène, un radical triméthylsilyloxy, dialcoylphosphoryloxy ou un radical de formule générale:

$- O\,SO_2\,R_3$

ou $- O\,CO\,R_4$

dans laquelle $R_3$ est un radical alcoyle, trifluorométhyle, trichlorométhyle ou un radical phényle éventuellement substitué par un atome d'halogène ou par un radical alcoyle ou nitro, et $R_4$ est défini comme $R_3$ ou repréaente un radical alcoylcarbonylméthyle, alcoylcarbonyl-2 éthyle, alcoyloxycarbonylméthyle, alcoyloxycarbonyl-2 éthyle ou alcoyloxy, les portions ou radicaux alcoyles cités ci-dessus étant (sauf mention spéciale) droits ou ramifiés et contenant 1 à 4 atomes de carbone, le cas échéant sous ses formes isomères et leurs mélanges, ainsi que leurs sels d'addition avec les acides et leur sels métalliques ou leurs sels d'addition avec les bases azotées loraqu'ils existent.

2 - Procédé pour la préparation d'un produit selon la revendication 1, caractérisé en ce que l'on fait agir un produit de formule générale:

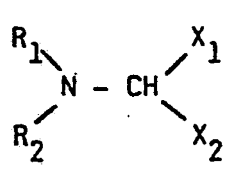

[[dans laquelle $R_1$ et $R_2$ sont des radicaux alcoyle contenant 1 à 4 atomes de carbone ou forment ensemble avec l'atome d'azote auquel ils sont rattachés, un hétérocycle à 5 ou 6 chaînons contenant éventuellement un autre hétéroatome choisi parmi l'oxygène, l'azote ou le soufre et éventuellement substitué par un radical alcoyle et,

$X_1$ et $X_2$, identiques ou différents, représentent un radical alcoyloxy ou un radical amino substitué défini comme $-NR_1R_2$ ci-dessus]] sur un produit de formule générale:

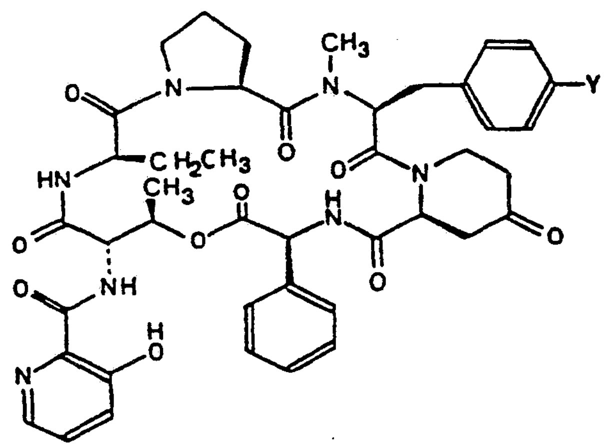

dans laquelle Y est défini comme à la revendication 1 pour obtenir un produit selon la revendication 1 pour lequel $R_1$ et $R_2$ sont définis comme ci-dessus, c'est-à-dire un produit de formule générale:

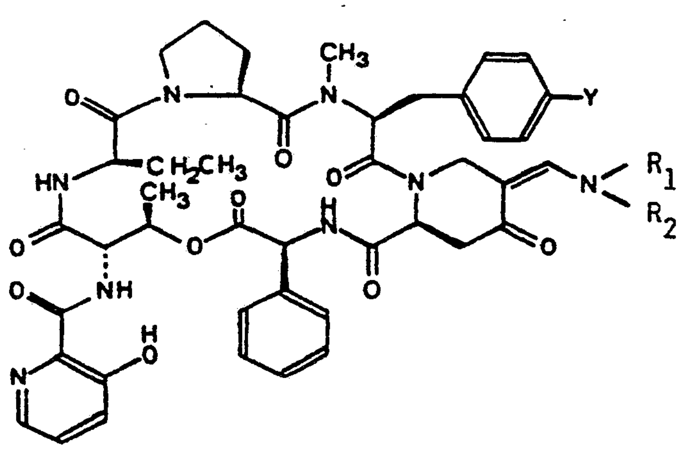

puis éventuellement,

1°) - on fait agir un borohydrure alcalin en présence d'un acide organique fort pour obtenir un produit selon la revendication 1 dans la formule duquel R représente un atome d'hydrogène, ou bien

2°) - on fait agir une amine de formule générale:

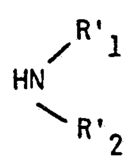

dans laquelle $R'_1$ et $R'_2$ ont la définition donnée à la revendication 1 pour $R_1$ et $R_2$ à l'exception des significations définies ci-dessus pour obtenir un produit selon la revendication 1 dans la formule duquel R est défini comme $-NR'_1R'_2$, ou bien

3') - on met en oeuvre une hydrolyse pour obtenir un produit de formule générale:

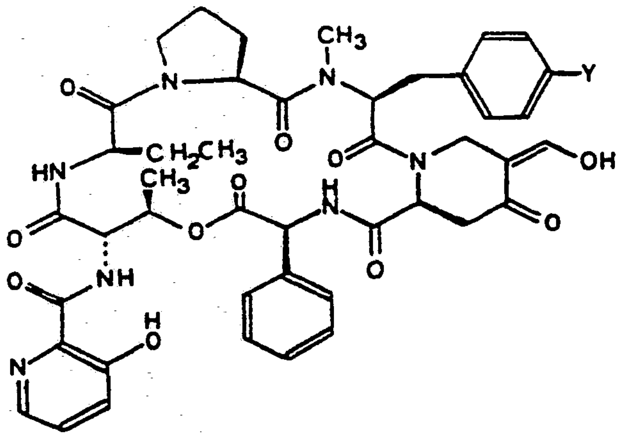

dans laquelle Y est défini comme dans la revendication 1, puis éventuellement de la transformation en un produit selon la revendication 1 pour lequel R est défini comme dans la revendication 1 en c), par action d'un agent d'halogénation ou d'un produit de formule générale:

R'-X

dans laquelle R' est un radical triméthylsilyloxy dialcoylphosphoryloxy ou un radical $-OSO_2R_3$ ou $-OCOR_4$ tel que défini dans la revendication 1, et X représente un atome d'halogène, puis lorsqu'ils existent sépare éventuellement le produit obtenu en ses isomères et transforme éventuellement le produit en un sel.

**Revendication**

pur l'Etat contractant AT

Un procédé de préparation de nouveaux dérivés de synergistines de formule générale

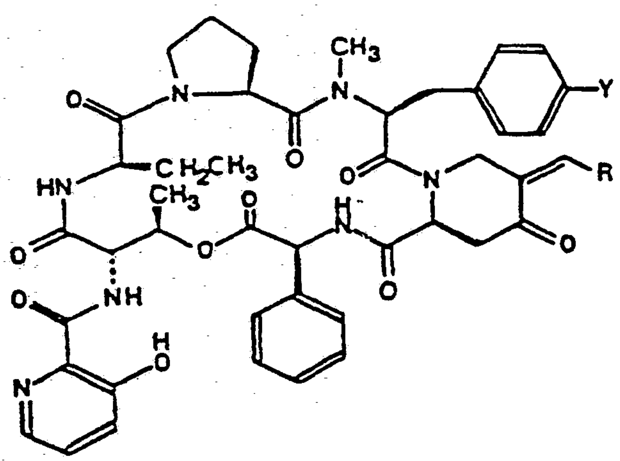

(I)

dans laquelle Y représente un atome d'hydrogène ou un radical diméthylamino et R représente:

a) soit un atome d'hydrogène ou un radical hydroxy,

b) soit un radical de formule générale:

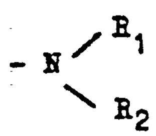

dans laquelle $R_1$ et $R_2$, identiques ou différents, représentent un atome d'hydrogène ou un radical phényle ou pyridyle (éventuellement substitués par un radical dialcoylamino dont la partie alcoyle contient 1 à 4 atomes de carbone en chaîne droite ou ramifiée) ou un radical alcoyle contenent 1 à 10 atomes de carbone en chaîne droite ou ramifiée éventuellenent substitué [par un radical hydroxy, mercapto, carboxy, pyridyle, anilino,

alcoylamino ou dialcoylamino dont au moins l'une des parties alcoyle est elle-même substituée par un radical hydroxy, mercapto, carboxy ou anilino], ou un radical alcényle

contenant 3 ou 4 atomes de carbone, ou un radical alcynyle contenant 3 ou 4 atomes de carbone, ou bien $R_1$ et $R_2$ forment ensemble avec l'atome d'azote auquel ils sont liés un hétérocycle à 5 ou 6 chaînons contenant éventuellement un autre hétéroatome choisi parmi l'oxygène, le soufre ou l'azote (éventuellement substitué par un radical alcoyle),

c) soit un atome d'halogène, un radical triméthylsilyloxy, dialcoylphosphoryloxy ou un radical de formule générale:

$-O\,SO_2\,R_3$

ou $-O\,CO\,R_4$

dans laquelle $R_3$ est un radical alcoyle, trifluorométhyle, trichlorométhyle ou un radical phényle éventuellement substitué par un atome d'halogène ou par un radical alcoyle ou nitro, et $R_4$ est défini comme $R_3$ ou représente un radical alcoylcarbonylméthyle, alcoylcarbonyl-2 éthyle, alcoyloxycarbonylméthyle, alcoyloxycarbonyl-2 éthyle ou alcoyloxy, les portions ou radicaux alcoyles cités ci-dessus étant (sauf mention spéciale) droits ou ramifiés et contenant 1 à 4 atomes de carbone, le cas échéant sous ses formes isomères et leurs mélanges. ainsi que de leurs sels d'addition avec les acides et leurs sels métalliques ou leurs sels d'addition avec les bases azotées lorsqu'ils existent,

caractérisé en ce que l'on fait agir un produit de formule générale:

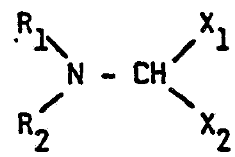

[[dans laquelle $R_1$ et $R_2$ sont des radicaux alcoyle contenant 1 à 4 atomes de carbone ou forment ensemble avec l'atome d'azote auquel ils sont rattachés, un hétérocycle à 5 ou 6 chaînons contenant éventuellement un autre hétéroatome choisi parmi l'oxygène, l'azote ou le soufre et éventuellement substitué par un radical alcoyle et, $X_1$ et $X_2$, identiques ou différents, représentent un radical alcoyloxy ou un radical amino substitué défini comme $-NR_1R_2$ ci-dessus]) sur un produit de formule générale:

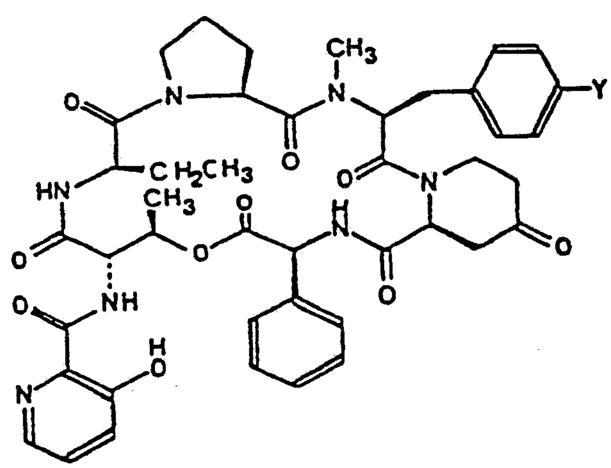

dans laquelle Y est défini comme précédemment pour obtenir un produit pour lequel $R_1$ et $R_2$ sont définis comme ci-dessus, c'est-à-dire un produit de formule générale:

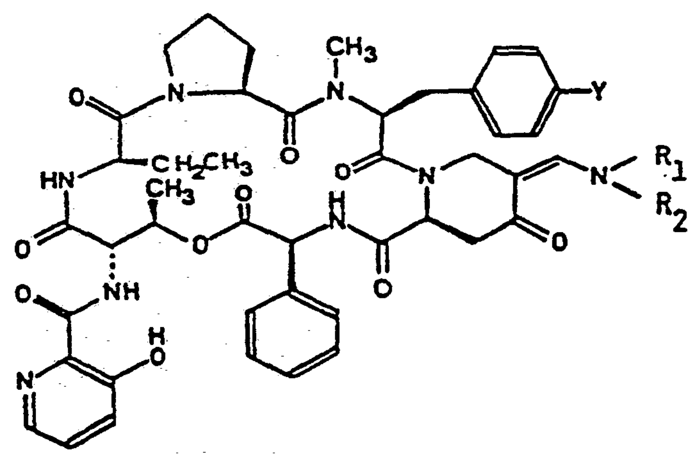

puis éventuellement,

1°) on fait agir un borohydrure alcalin en présence d'un acide organique fort pour obtenir un produit de formule générale (I) pour lequel R représente un atome d'hydrogène, ou bien

2°) on fait agir une amine de formule générale:

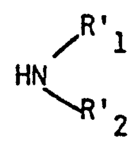

dans laquelle $R'_1$ et $R'_2$ ont la définition donnée pour $R_1$ et $R_2$ dans la formule générale (I) à l'exception des significations définies ci-dessus pour obtenir un produit dans la formule duquel R est défini comme $-NR'_1R'_2$, ou bien

3°) on met en oeuvre une hydrolyse pour obtenir un produit de formule générale:

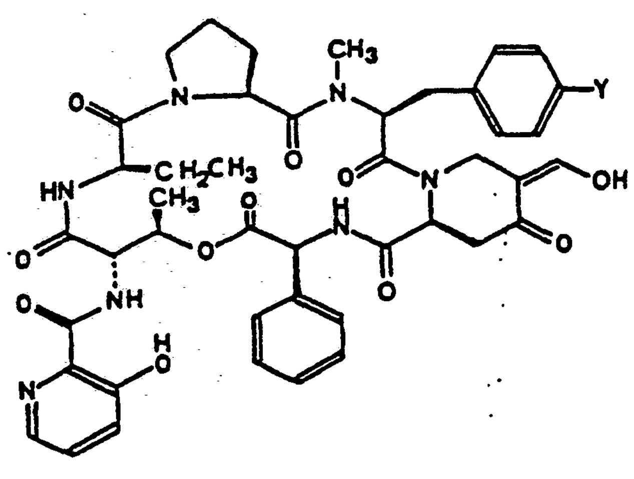

dans laquelle Y est défini comme précédemment, puis éventuellement transforme le produit obtenu en un produit de formule générale (I) pour lequel R est défini comme précédemment en c), par action d'un agent d'halogénation ou d'un produit de formule générale:

R'-X

dans laquelle R est un radical triméthylsilyloxy, diacloylphosphoryloxy ou un radical $-OSO_2R_3$ ou $-OCOR_4$ tel que défini précédemment, et X représente un atome d'halogène, puis lorsqu'ils exietent sépare éventuellement le produit obtenu en ses isomères et transforme éventuellement le produit en un sel.

**Patentansprüche**

für die Vertragsstaaten BE, CH, DE, FR, GB, IT, LI, LU, NL, SE

Neues Synergistinderivat, dadurch gekennzeichnet, daß es der allgemeinen Formel

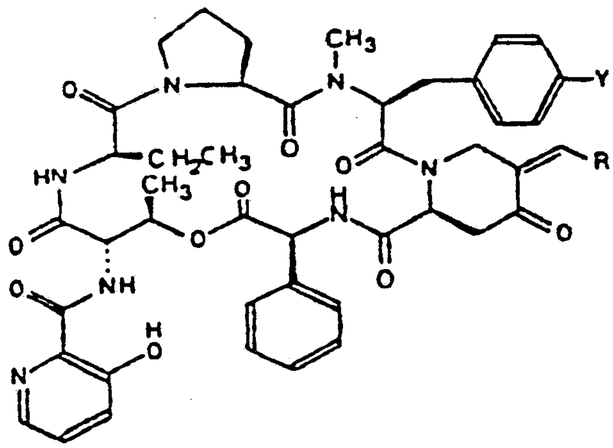

entspricht, worin Y ein Wasserstoffatom oder einen Dimethylaminorest bedeutet, und R bedeutet:.

a) entweder ein Wasserstoffatom oder einen Hydroxyrest,

b) oder einen Rest der allgemeinen Formel

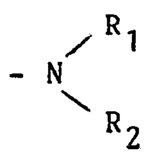

worin $R_1$ und $R_2$, die identisch oder verschieden sind, ein Wasserstoffatom oder einen Phenyl- oder Pyridylrest (gegebenenfalls substituiert durch einen Dialkylaminorest, dessen Alkylteil 1 bis 4 Kohlenstoffatome in gerader oder verzweigter Kette enthält) oder einen Alkylrest mit 1 bis 10 Kohlenstoffatomen in gerader oder verzweigter Kette, gegebenenfalls substituiert durch einen Rest Hydroxy, Mercapto, Carboxy, Pyridyl, Anilino, Alkylamino oder Dialkylamino, wovon wenigstens einer der Alkylteile selbst substituiert ist durch einen Hydroxy-, Mercapto-, Carboxy- oder Anilinorest] , oder einen Alkenylrest mit 3 oder 4 Kohlenstoffatomen oder einen Alkinylrest mit 3 oder 4 Kohlenstoffatomen bedeutet, oder aber $R_1$ und $R_2$ bilden zusammen mit dem Stickstoffatom, an das sie gebunden sind, einen Heterocyclus mit 5 oder 6 Kettengliedern, der gegebenenfalls ein weiteres Heteroatom, ausgewählt unter Sauerstoff, Schwefel oder Stickstoff enthält (gegebenenfalls substituiert durch einen Alkylrest),

c) oder ein Halogenatom, einen Trimethylsilyloxyrest, Dialkylphosphoryloxyrest oder einen Rest der allgemeinen Formel

$- O\ SO_2\ R_3$

oder $-O\ CO\ R^4$

bedeutet, worin $R_3$ ein Alkyl-, Trifluormethyl-, Trichlormethyloder ein Phenylrest ist, gegebenenfalls substituiert durch ein Halogenatom oder durch einen Alkyl- oder Nitrorest und $R_4$ ist wie $R_3$ definiert oder bedeutet einen Rest Alkylcarbonylmethyl, 2-Alkylcarbonylethyl, Alkoxycarbonylmethyl, 2-Alkyloxycarbonylethyl oder Alkyloxy, wobei die oben erwähnten Alkylteile oder -reste (falls nichts anderes angegeben ist) gerade oder verzweigt sind und 1 bis 4 Kohlenstoffatome enthalten, gegebenenfalls in ihren isomeren Formen und deren Gemischen sowie ihre Additionssalze mit Säuren und ihre Metallsalze oder ihre Additionssalze mit Stickstoffbasen, falls sie existieren.

2. Verfahren zur Herstellung eines Produkts gemäß Anspruch 1, dadurch gekennzeichnet, daß man ein Produkt der allgemeinen Formel

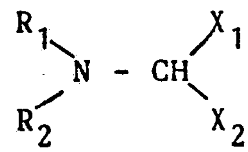

[[worin $R_1$ und $R_2$ Alkylreste mit 1 bis 4 Kohlenstoffatomen sind oder zusammen mit dem Stickstoffatom, an das sie gebunden sind, einen Heterocyclus mit 5 oder 6 Kettengliedern bilden, der gegebenenfalls ein weiteres Heteroatom, ausgewählt unter Sauerstoff, Stickstoff oder Schwefel, enthält, und gegebenenfalls durch einen Alkylrest substituiert ist, und $X_1$ und $X_2$, die identisch oder verschieden sind, bedeuten einen Alkyloxyrest oder einen substituierten Aminorest, wie oben bei $-NR_1R_2$ definiert]] auf ein Produkt der allgemeinen Formel

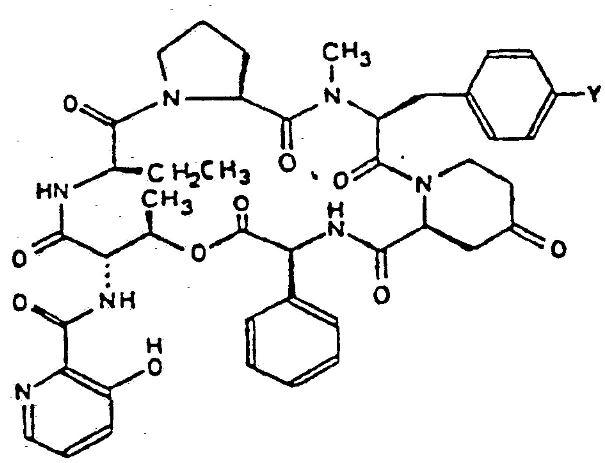

worin Y wie in Anspruch 1 definiert ist, einwirken läßt, um ein Produkt gemäß Anspruch 1 zu erhalten, für das $R_1$ und $R_2$ wie vorstehend definiert sind, d.h. ein Produkt der allgemeinen Formel

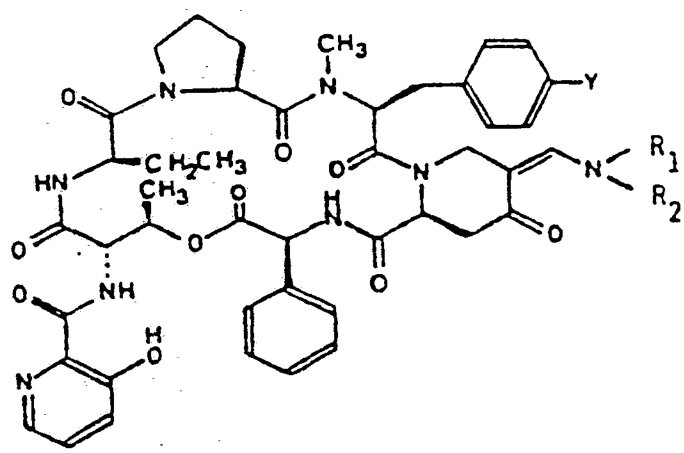

und daß man gegebenenfalls

1.) ein Alkaliborhydrid in Gegenwart einer starken organischen Säure einwirken läßt, um ein Produkt gemäß Anspruch 1 zu erhalten, in dessen Formel R ein Wasserstoffatom bedeutet, oder daß man

2.) ein Amin der allgemeinen Formel

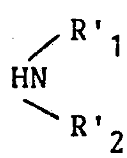

worin $R'_1$ und $R'_2$ die in Anspruch 1 für $R_1$ und $R_2$ angegebene Definition haben mit Ausnahme der oben definierten Bedeutungen, einwirken läßt, um ein Produkt gemäß Anspruch 1 zu erhalten, in dessen Formel R

wie $-NR'_1R'_2$ definiert ist, oder daß man

3.) eine Hydrolyse bewirkt, um ein Produkt der allgemeinen Formel

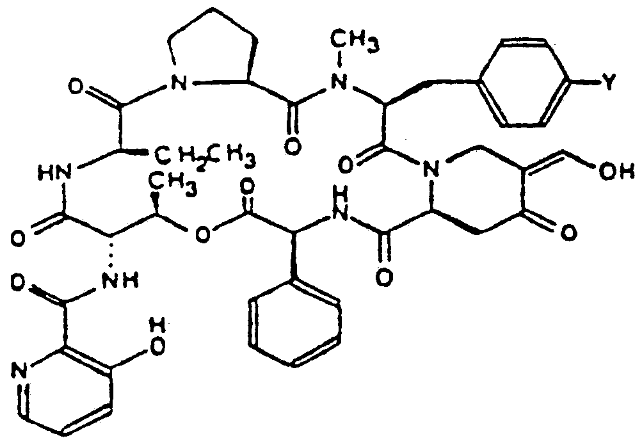

worin Y wie in Anspruch 1 definiert ist, zu erhalten, und dann gegebenenfalls zur Umwandlung in ein Produkt gemäß Anspruch 1 für das R wie in Anspruch 1 in c) definiert ist, durch Einwirkung eines Halogenierungsmittels oder eines Produkts der allgemeinen Formel

R'-X

worin R' ein Trimethylsilyloxy-, Dialkylphosphoryloxyrest oder ein Rest $-OSO_2R_3$ oder $-OCOR_4$ ist, wie in Anspruch 1 definiert, und X ein Halogenatom bedeutet, und daß man dann, falls sie existieren, gegebenenfalls das erhaltene Produkt in seinen Isomeren trennt und das Produkt gegebenenfalls in ein Salz überführt.

**Patentanspruch**

für den Vertragsstaat AT:

Ein Verfahren zur Herstellung neuer Synergistinderivate der allgemeinen Formel:

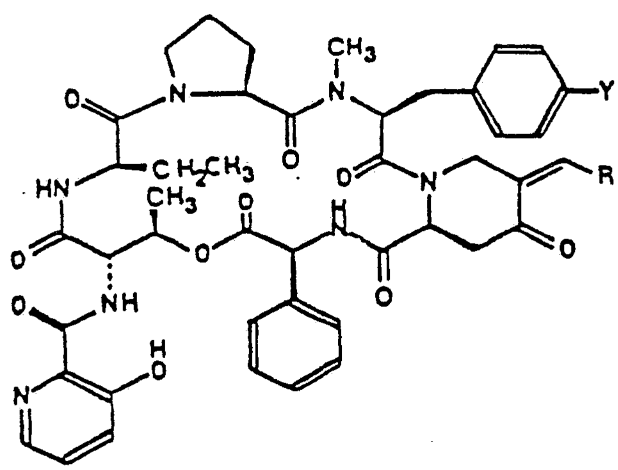

(I)

in welcher Y ein Wasserstoffatom oder einen Dimethylaminorest darstellt und R darstellt:

a) entweder ein Wasserstoffatom oder einen Hydroxyrest,

b) oder einen Rest der allgemeinen Formel:

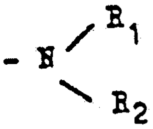

in welcher $R_1$ und $R_2$ unabhängig voneinander ein Wasserstoff atom oder einen Phenyl- oder Pyridylrest (gegebenenfalls substituiert durch einen Dialkylaminorest, dessen

Alkylteil 1 bis 4 Kohlenstoffatome in gerader oder verzweigter Kette enthält), oder einen geradkettigen oder verzweigten Alkylrest mit 1 bis 10 Kohlenstoffatomen, gegebenenfalls substituiert (durch einen Hydroxy-, Mercapto-, Carboxy-, Pyridyl-, Anilin-, Alkylamino- oder Dialkylaminorest, worin zumindest einer der Alkylteile seinerseits durch einen Hydroxy-, Mercapto-, Carboxy- oder Anilinrest substituiert ist] oder einen Alkenylrest mit 3 oder 4 Kohlenstoffatomen, einen Alkynylrest mit 3 oder 4 Kohlenstoffatomen darstellen oder $R_1$ und $R_2$ zusammen mit dem Stickstoffatom, an das sie gebunden sind, einen Heterocyclus mit 5 oder 6 Gliedern bilden, der gegebenenfalls ein weiteres Heteroatom, ausgewählt aus Sauerstoff, Schwefel oder Stickstoff, enthält (und gegebenenfalls durch einen Alkylrest substituiert ist)

c) oder ein Halogenatom, einen Trimethylsilyloxy-, Dialkylphosphoryloxyrest oder einen Rest der allgemeinen Formel:

$-O\,SO_2\,R_3$

oder $-O\,CO\,R_4$

darstellt, in welcher $R_3$ ein Alkyl-, Trifluormethyl-, Trichlormethylrest oder Phenylrest (gegebenenfalls substituiert durch ein Halogenatom oder durch einen Alkyl- oder Nitrorest, ist und $R_4$ die Bedeutung von $R_3$ hat oder ein Alkylcarbonylmethyl-, 2-Alkylcarbonyläthyl-, Alkyloxycarbonylmethyl-, 2-Alkyloxycar-bonyläthyl- oder Alkyloxyrest ist, wobei die genannten Alkylteile oder -reste (ohne besondere Erwähnung) geradkettig oder verzweigt sind und 1 bis 4 Kohlenstoffatome enthalten, zutreffendenfalls in ihren isomeren Formen und deren Mischungen sowie von ihren Säureadditionssalzen und ihren Metallsalzen oder ihren Additionssalzen mit Stickstoffbasen, falls es diese gibt, dadurch gekennzeichnet, daß man eine Verbindung der allgemeinen Formel:

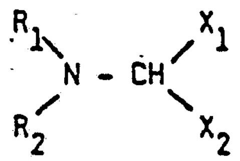

(in welcher $R_1$ und $R_2$ Alkylreste mit 1 bis 4 Kohlenstoffatomen sind oder zusammen mit dem Stickstoffatom, an das sie gebunden sind, einen Heterocyclus mit 5 oder 6 Gliedern bilden, der gegebenenfalls ein weiteres Heteroatom, ausgewählt aus Sauerstoff, Stickstoff oder Schwefel, enthält und gegebenenfalls durch einen Alkylrest substituiert ist, und $X_1$ und $X_2$ unabhängig voneinander einen Alkyloxyrest oder einen substituierten Aminorest mit der obigen Bedeutung von $-NR_1R_2$ darstellen) mit einer Verbindung der allgemeinen Formel:

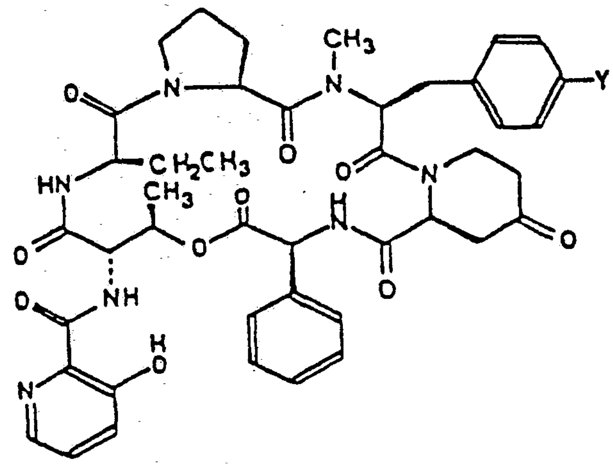

in welcher Y die obige bedeutung hat, umsetzt unter Bildung einer Verbindung, worin $R_1$ und $R_2$ die obige Bedeutung haben, d.h. einer Verbindung der allgemeinen Formel:

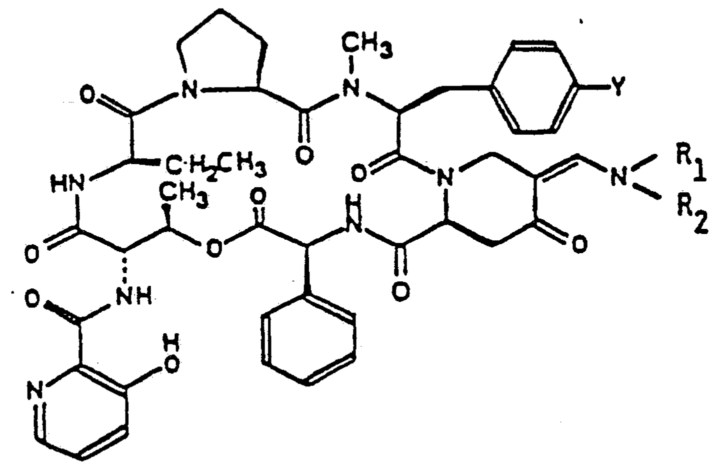

und daß man dann gegebenenfalls:

1°) ein Alkaliborhydrid in Gegenwart einer starken organischen Säure einwirken läßt unter Bildung einer Verbindung der allgemeinen Formel (I), worin R ein Wasserstoffatom darstellt, oder

2°) ein Amin der allgemeinen Formel:

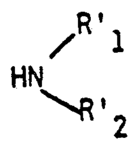

in welcher $R'_1$ und $R_2$ die für $R_1$ und $R_2$ in der allgemeinen Formel (I) angegebene Bedeutung, mit Ausnahme der obigen Bedeutungen, haben, einwirken läßt unter Bildung einer Verbindung, in deren Formel R die Bedeutung von $-NR'_1R'_2$ hat, oder

3°) eine Hydrolyse durchführt unter Bildung einer Verbindung der allgemeinen Formel:

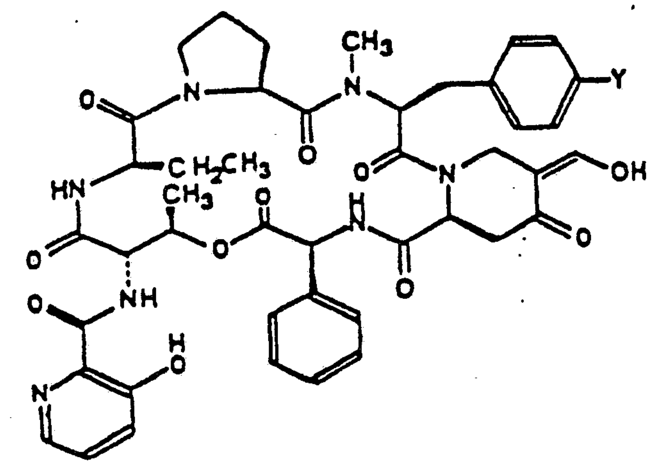

in welcher Y die obige Bedeutung hat, und daß man dann gegebenenfalls die erhaltene Verbindung in eine Verbindung der allgemeinen Formel (I), worin R die oben unter c) angegegebene Bedeutung hat, überführt durch Einwirkung eines Halogenierungsmittels oder einer Verbindung der allgemeinen Formel:

R'-X

in welcher R' ein Trimethylsilyloxy-, Dialkylphosphoryloxyrest oder ein Rest $-OSO_2R_3$ oder $-OCOR_4$ wie oben definiert, ist und X ein Halogenatom darstellt, und daß man dann - wenn es sie gibt - die erhaltene Verbindung in ihre Isomeren spaltet und die Verbindung gegebenenfalls in ein Salz umwandelt.

**Claims**

for the contracting States: BE CH DE FR GB IT LI LU NL SE

1. New synergistin derivative, characterized in that it corresponds to the general formula:

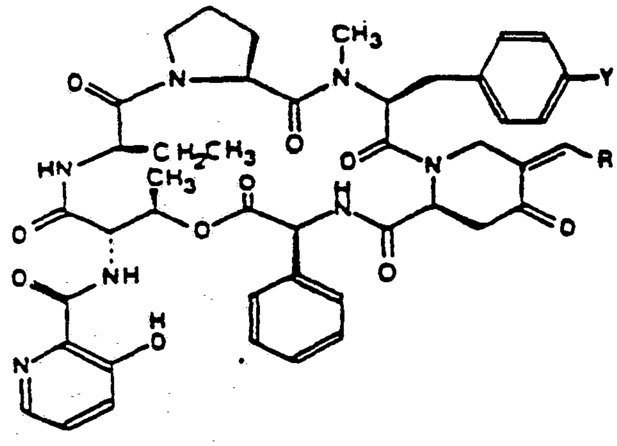

in which Y denotes a hydrogen atom or a dimethylamino radical and R denotes:
a) either a hydrogen atom or a hydroxy radical,
b) or a radical of general formula:

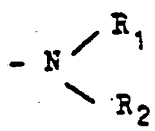

in which $R_1$ and $R_2$, which are identical or different, denote a hydrogen atom or a phenyl or pyridyl radical (which are optionally substituted by a dialkylamino radical whose alkyl moiety contains 1 to 4 carbon atoms as a straight or branched chain) or an alkyl radical containing 1 to 10 carbon atoms as a straight or branched chain, optionally substituted (by a hydroxy, mercapto, carboxy, pyridyl, anilino, alkylamino or dialkylamino radical in which at least one of the alkyl moieties is itself substituted by a hydroxy, mercapto, carboxy or anilino radical), or an alkenyl radical containing 3 or 4 carbon atoms, or an alkynyl radical containing 3 or 4 carbon atoms, or else $R_1$ and $R_2$ form, together with the nitrogen atom to which they are attached, a 5- or 6-membered heterocyclic ring optionally containing another heteroatom chosen from oxygen, sulphur or nitrogen (optionally substituted by an alkyl radical),

c) or a halogen atom, a trimethylsiloxy or dialkylphosphoryloxy radical, or a radical of general formula:
$- O\ SO_2\ R_3$
or $- O\ CO\ R_4$
in which $R_3$ is an alkyl, trifluoromethyl or trichloromethyl radical or a phenyl radical optionally substituted by a halogen atom or by an alkyl or nitro radical, and $R_4$ is defined as $R_3$ or denotes an alkylcarbonylmethyl, 2-alkyl-carbonylethyl, alkyloxycarbonylmethyl, 2-alkyloxycarbonyl-ethyl or alkyloxy radical, the alkyl moieties or radicals mentioned above being (unless expressly mentioned) straight or branched and containing 1 to 4 carbon atoms, where appropriate in the form of its isomers and mixtures thereof, as well as their salts of addition to acids and their metal salts or their salts of addition to nitrogenous bases where such exist.

2. Process for the preparation of a product according to Claim 1, characterized in that a product of general formula:

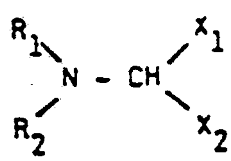

(in which $R_1$ and $R_2$ are alkyl radicals containing 1 to 4 carbon atoms or form, together with the nitrogen atom to which they are attached, a 5- or 6-membered heterocyclic ring optionally containing another heteroatom chosen from oxygen, nitrogen or sulphur and optionally substituted by an alkyl radical, and $X_1$ and $X_2$, which are identical or different, denote an alkoxy radical or a substituted amino radical defined as $-NR_1R_2$ above) is reacted with a product of general formula:

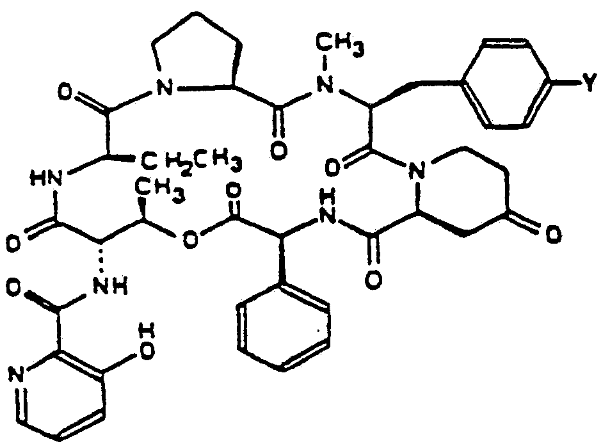

in which Y is defined as in Claim 1, to obtain a product according to Claim 1 for which $R_1$ and $R_2$ are defined as above, that is to say a product of general formula:

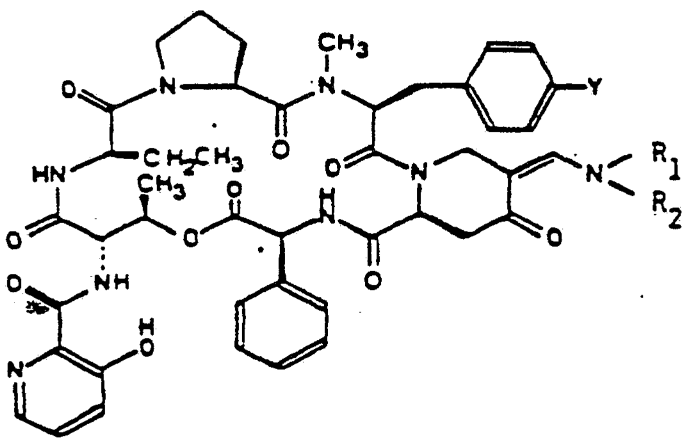

and then, if appropriate,

1) an alkali metal borohydride is reacted in the presence of a strong organic acid to obtain a product according to Claim 1, in the formula of which R denotes a hydrogen atom, or

2) an amine of general formula:

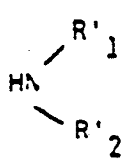

in which $R'_1$ and $R'_2$ have the definition given for $R_1$ and $R_2$ in Claim 1, except for the meanings defined above, is reacted to obtain a product according to Claim 1 in the formula of which R is defined as $-NR'_1R'_2$, or

3) a hydrolysis is carried out to obtain a product of general formula:

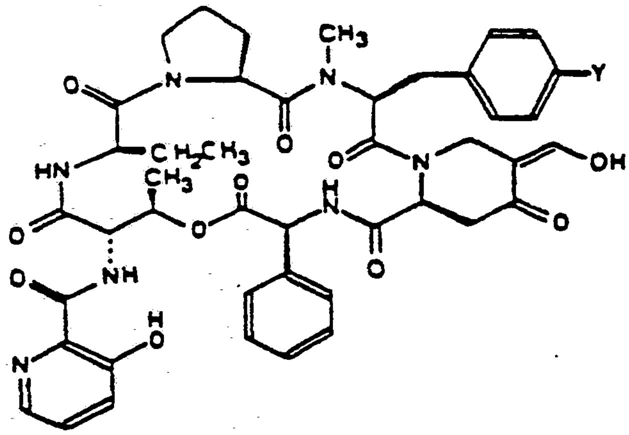

in which Y is defined as in Claim 1, and then, if appropriate, conversion to a product according to Claim 1, in which R is defined as in Claim 1c), is effected by the action of a halogenating agent or of a product of general formula:

R'-X

in which R' is a trimethylsiloxy or dialkylphosphoryloxy radical or a radical $-OSO_2R_3$ or $-OCOR_4$, as defined in Claim 1, and X denotes a halogen atom, and then the product obtained is separated, if appropriate, into its isomers, where such exist, and the product is optionally converted into a salt.

**Claim**

for the contracting State AT

A process for the preparation of new synergistin derivatives, of general formula:

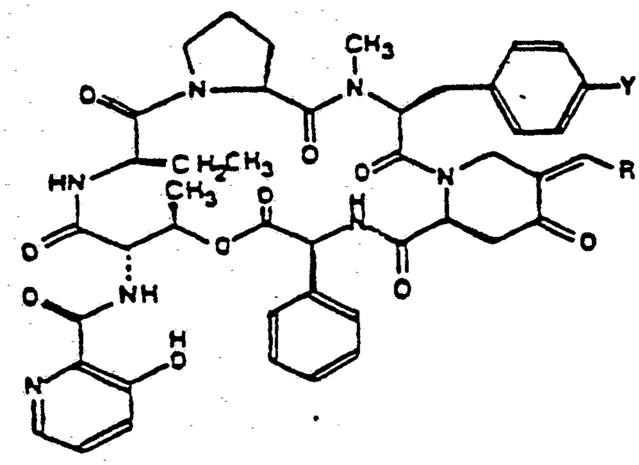

(I)

in which Y denotes a hydrogen atom or a dimethylamino radical and R denotes:

a) either a hydrogen atom or a hydroxy radical,

b) or a radical of general formula:

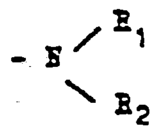

in which $R_1$ and $R_2$, which are identical or different, denote a hydrogen atom or a phenyl or pyridyl radical (which are optionally substituted by a dialkylamino radical whose alkyl moiety contains 1 to 4 carbon atoms as a straight or branched chain) or an alkyl radical containing 1 to 10 carbon atoms as a straight or branched chain, optionally substituted (by a hydroxy, mercapto, carboxy, pyridyl, anilino, alkylamino or dialkylamino

radical in which at least one of the alkyl moieties is itself substituted by a hydroxy, mercapto, carboxy or anilino radical), or an alkenyl radical containing 3 or 4 carbon atoms, or an alkynyl radical containing 3 or 4 carbon atoms, or else $R_1$ and $R_2$ form, together with the nitrogen atom to which they are attached, a 5- or 6-membered heterocyclic ring optionally containing another heteroatom chosen from oxygen, sulphur or nitrogen (optionally substituted by an alkyl radical),

c) or a halogen atom, a trimethylsiloxy or dialkylphosphoryloxy radical or a radical of general formula:

$-O\ SO_2\ R_3$

or $-O\ CO\ R_4$

in which $R_3$ is an alkyl, trifluoromethyl or trichloromethyl radical or a phenyl radical optionally substituted by a halogen atom or by an alkyl or nitro radical, and $R_4$ is defined as $R_3$ or denotes an alkylcarbonylmethyl, 2-alkyl-carbonylethyl, alkyloxycarbonylmethyl, 2-alkyloxycarbonyl-ethyl or alkyloxy radical, the alkyl moieties or radicals mentioned above being (unless expressly mentioned) straight or branched and containing 1 to 4 carbon atoms, where appropriate in the form of its isomers and mixtures thereof, as well as their salts of addition to acids and their metal salts or their salts of addition to nitrogenous bases where such exist, characterized in that a product of general formula:

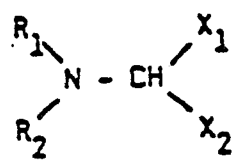

(in which $R_1$ and $R_2$ are alkyl radicals containing 1 to 4 carbon atoms or form, together with the nitrogen atom to which they are attached, a 5- or 6-membered heterocyclic ring optionally containing another heteroatom chosen from oxygen, nitrogen or sulphur and optionally substituted by an alkyl radical, and $X_1$ and $X_2$, which are identical or different, denote an alkoxy radical or a substituted amino radical defined as $-NR_1R_2$ above) is reacted with a product of general formula:

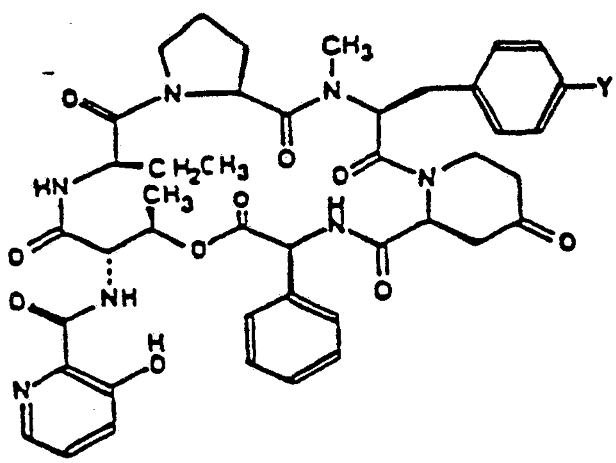

in which Y is defined as previously to obtain a product for which $R_1$ and $R_2$ are defined as above, that is to say a product of general formula:

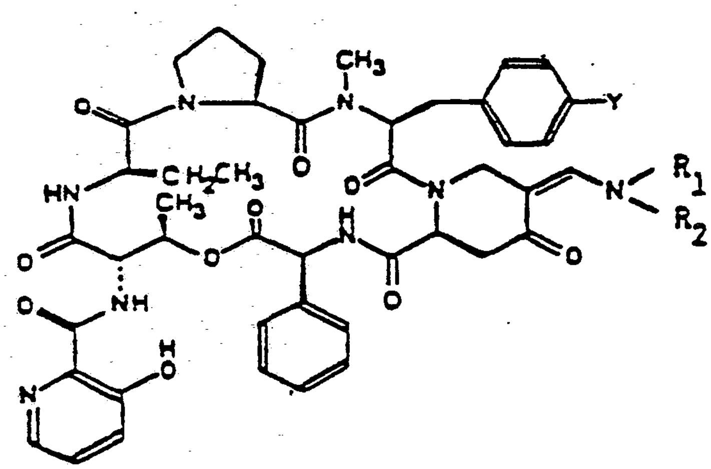

and then, if appropriate,

1) an alkali metal borohydride is reacted in the presence of a strong organic acid to obtain a product of general formula (I) for which R denotes a hydrogen atom, or

2) an amine of general formula:

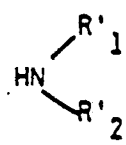

in which $R'_1$ and $R'_2$ have the definition given for $R_1$.and $R_2$ in the general formula (I), except for the meanings defined above, is reacted to obtain a product in the formula of which R is defined as $-NR'_1R'_2$, or

3) a hydrolysis is carried out to obtain a product of general formula:

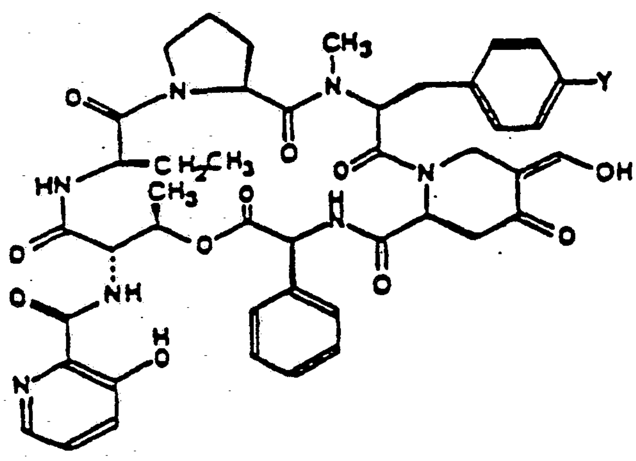

in which Y is defined as before, and then, if appropriate, the product obtained is converted to a product of general formula (I), for which R is defined as previously in c), by the action of a halogenating agent or of a product of general formula:.

R'-X

in which R' is a trimethylsiloxy or dialkylphosphoryloxy radical or a radical $-OSO_2R_3$ or $-DCOR_4$, as defined previously, and X denotes a halogen atom, and then the product obtained is separated, if appropriate, into its isomers, where such exist, and the product is optionally converted into a salt.